# EUROPEAN PATENT APPLICATION

(11) **EP 2 824 171 A1**
(43) Date of publication of application: **14.01.2015**
(21) Application number: 13757236.8
(22) Date of filing: 05.03.2013
(51) Int. Cl.: C12M 1/00, C12Q 1/68

(54) **HIGH SPEED GENE AMPLIFICATION DETECTION DEVICE**

(30) Priority: 06.03.2012 JP 2012049185
(71) Applicant: Kanagawa Academy Of Science And Technology, Kawasaki-shi, Kanagawa 213-0012 (JP); On-Chip Cellomics Consortium, Tokyo 100-0005 (JP); National University Corporation Tokyo Medical and Dental University, Bunkyo-ku Tokyo 113-8510 (JP)
(72) Inventor: YASUDA Kenji, Tokyo 113-8510 (JP); TERAZONO Hideyuki, Kawasaki-shi Kanagawa 213-0012 (JP); KIM Hyonchol, Kawasaki-shi Kanagawa 213-0012 (JP); HATTORI Akihiro, Tokyo 100-0005 (JP)
(74) Representative: Prinz & Partner mbB
(86) International application number: PCT/JP2013/055912
(87) International publication number: WO 2013/133244

(57) **Abstract**

The present invention provides a high-speed gene amplification device including an additional mechanism that allows more stable temperature control, a pre-treatment mechanism that performs pre-treatment including a pre-PCR reaction reverse transcription reaction process that allows RNA detection, a melting curve analysis function, chip technology optimal for holding liquid droplets and performing optical measurements and an optical measurement function for a PCR reaction.

## Description

### TECHNICAL FIELD

The present invention relates to a gene analysis device using a reaction container, which is suitable for rapidly performing an analysis with a small amount of gene for studies or clinical practice in basic bioscience, basic medical research and medical settings, and specifically the present invention relates to, for example, a gene analysis using a reaction device for detecting a particular nucleotide sequence at high speed from a nucleic-acid base sequence such as genomic DNA, messenger RNA or the like derived from an animal including a human or a plant.

### BACKGROUND ART

Polymerase chain reaction (hereinafter, abbreviated as PCR) is a method for amplifying a particular nucleotide sequence from a mixture of various types of nucleic acids. A particular nucleic acid sequence can be amplified by performing at least one cycle of the following steps: the step of adding, into the mixture, a DNA template such as, for example, genomic DNA or complementary DNA obtained by reverse transcription from messenger RNA, two or more types of primers, thermostable enzymes, salt such as magnesium or the like, and four types of deoxyribonucleoside triphosphates (dATP, dCTP, dGTP and dTTP), and splitting the nucleic acids; the step of binding the primers into the nucleic acids; and the step of allowing hybridization using, as a template, the nucleic acids bound by the primers and the thermostable enzymes. Thermal cycling is performed by increasing and decreasing the temperature of a reaction container used for DNA amplification reaction. There are various mechanisms for changing the temperature, including a mechanism in which the temperature of the reaction container containing a sample is changed through heat exchange using a heater, a Peltier element or hot air; a mechanism in which the temperature is changed by alternately bringing the reaction container into contact with heater blocks or liquid baths of different temperatures; and a method by which the temperature is changed by running a sample through a flow channel that has regions of different temperatures. Currently, the fastest commercially available device is, for example, Light Cycler from Roche, which has a mechanism where a sample, DNA polymerase, small sections of DNA as primers and a fluorescent dye label for measurement are placed into each of a plurality of glass capillary tubes, and the temperatures of small amounts of liquid droplets in the capillary tubes are changed by blowing hot air at a temperature intended for the liquid droplets, for example, at two temperatures of 55°C and 95°C, while at the same time, the glass capillary tubes are irradiated with fluorescent dye-exciting light to measure the resulting fluorescence intensity. By any of these methods, the temperature of the sample can be repeatedly changed.

A fluid impingement thermal cycler device has been reported that controls the temperature of a sample by impingement of fluid jet on an outer wall of a sample-containing region (Japanese PCT National Phase Laid-Open Patent Publication No. 2001-519224 (Patent Document 1)). In order to realize PCR performed at higher speed, the present inventors have so far developed a technology of irradiating water with infrared rays of a wavelength that has a specific absorbance in water to change the temperature of minute water droplets at high speed, and also an ultra-high PCR device capable of performing temperature cycling at ultra-high speed by use of circulating water, more specifically, through heat exchange with circulating water (Japanese Laid-Open Patent Publication No. 2008-278791, Japanese Laid-Open Patent Publication No. 2009-118798, WO2010/113990 and WO2011/105507) (Patent Documents Nos. 2-5)).

### CITATION LIST

### PATENT LITERATURE

Patent Document 1: Japanese PCT National Phase Laid-Open Patent Publication No. 2001-519224
Patent Document 2: Japanese Laid-Open Patent Publication No. 2008-278791
Patent Document 3: Japanese Laid-Open Patent Publication No. 2009-118798
Patent Document 4: WO2010/113990
Patent Document 5: WO2011/105507

### SUMMARY OF INVENTION

### TECHNICAL PROBLEM

When an operation cycle is to be repeated between two or more temperatures with a rapid temperature change as described above, it is difficult for the conventional technologies to 1) control the temperature strictly, 2) maintain the temperature stably, and 3) avoid overshoot during the transition to a target temperature. For example, the temperature change rate obtained with a heater or a Peltier element is as slow as about a few degrees Celsius per second. When the temperature is to be changed to the target temperature at high speed through the relationship between the heat generation and the heat conduction, it is difficult to avoid overshoot in the temperature. In addition, basically, when heat conduction through a solid substance is utilized, a heat gradient is generated between the heat source and the surface thereof, which renders strict control on the temperature impossible. Furthermore, since heat is lost at the moment when the sample touches the heater or the Peltier element, the surface restores a predetermined temperature with delay. In the case where a reaction vessel is to be brought into contact with a plurality of different heaters or liquid baths, the transfer mechanism is complicated and it is difficult to control the temperature of the heaters or liquid baths. With a method by which a sample is run through a flow channel having regions of different temperatures, a problems arises that the surface temperature of the flow channel itself changes with the movement of the sample, and thus it is difficult to control the temperature. In the case where the temperature is to be changed by blowing hot air, a large amount of air needs to be blown because the heat capacity of the air is small. Such a small heat capacity of the air makes it difficult to strictly control the eventual temperature of the air, blown by use of an electrically-heated wire or the like, in increments of 1°C.

So far, the present inventors independently developed a reaction control device that is capable of constantly supplying energy to a target and conducting accurate temperature control, accurate temperature measurement, and rapid temperature increase and decrease by use of steady infrared irradiation or warm water that is refluxing at high speed for the purpose of supplying a constant amount of heat continuously. The present inventors have also combined a fluorescence detection system with the reaction control device to develop a high-speed PCR detection device that carries out a fluorescence detection method for detecting an amplification reaction of DNA by use of a fluorescent dye, the fluorescence intensity of which is increased along with the amplification of DNA by PCR (Patent Documents 2 through 5).

The present invention has an object of further improving the above-described conventional inventions made by the present inventors and thus providing a nucleic acid reaction control device capable of performing more accurate temperature control, more accurate temperature measurement, and more rapid temperature increase and decrease.

### SOLUTION TO PROBLEM

In light of the above-described object, the present invention provides a high-speed reaction control device (e.g., high-speed PCR device) including an additional mechanism that allows more stable temperature control, and a gene analysis or gene amplification method using the same.

Namely, the present invention provides the following device and method.
[1] A device for gene analysis or gene amplification, the device comprising:
   a reaction vessel including one or a plurality of wells for accommodating a sample liquid containing nucleic acid;
   a heat sink or a heat exchange chamber;
   a temperature controller, for the heat sink or the heat exchange chamber, that keeps a temperature of the heat sink or the heat exchange chamber at a first temperature;
   a thermoelectric element that is located between the heat sink or the heat exchange chamber and the reaction vessel and acts as a medium for heat transfer between the heat sink or the heat exchange chamber and the reaction vessel; and
   a thermoelectric element controller that changes a temperature of the reaction vessel between the first temperature and a second temperature by controlling an operation of the thermoelectric element;
   wherein when the thermoelectric element is in an off state, the temperature of the reaction vessel is the first temperature.
[2] The device according to [1], wherein:
   the first temperature is a nucleic acid elongation reaction temperature; and
   the second temperature is (i) a nucleic acid denaturation temperature or (ii) the nucleic acid denaturation temperature or a nucleic acid annealing temperature.
[3] The device according to [1] or [2], wherein the temperature controller includes:
   (i) a temperature sensor;
   (ii) a heating wire for heating the heat sink, and
   (iii) a feedback control mechanism that controls power supply to the heating wire based on temperature information on the heat sink from the temperature sensor.
[4] The device according to [1] or [2], wherein the temperature controller is a liquid reflux type temperature controller that controls the temperature of the heat exchange chamber by refluxing a liquid of a predetermined temperature between the heat exchange chamber and a liquid reservoir that is in fluid connection with the heat exchange chamber by a tubular flow channel and holds the liquid of the predetermined temperature.
[5] The device according to any one of [1] through [4], wherein the thermoelectric element controller includes:
   a temperature sensor; and
   a computer that controls an operation of the thermoelectric element based on temperature information on the reaction vessel from the temperature sensor.
[6] The device according to any one of [1] through [5], wherein the thermoelectric clement is a Peltier element.
[7] The device according to any one of [1] through [6], further comprising a heat conductive thin plate located between the thermoelectric element and the reaction vessel in order to raise a heat transfer efficiency between the thermoelectric element and the reaction vessel.
[8] The device according to any one of [1] through [7], wherein a bottom surface and a wall surface of the reaction vessel each have a thickness of 1 micrometer to 100 micrometers and are formed of a metal material selected from the group consisting of aluminum, nickel, magnesium, titanium, platinum, gold, silver and copper, or silicon.
[9] The device according to any one of [1] through [8], wherein an amount of the sample liquid is several tens of microliters per well.
[10] The device according to any one of [1] through [9], further comprising a fluorescence detector that, in the case where the sample liquid contains a fluorescence dye, detects fluorescence emitted by the fluorescent dye in the one or the plurality of wells in association with a switch of the temperature of the reaction vessel, and measures a time-wise change in fluorescence intensity.
[11] The device according to any one of [1] through [10], further comprising a reaction vessel casing that covers the reaction vessel in order to prevent evaporation of droplets of the sample liquid located in the one or the plurality of wells and includes a heat retention member for preventing dew condensation.
[12] The device according to [11], wherein the reaction vessel casing further includes an aperture or an optical window that facilitates measurement of an optical signal from the sample liquid in the reaction vessel casing; and the optical window is provided with an optically transparent heat generator.
[13] The device according to any one of [1] through [9], wherein a pillar is located at a position where the sample liquid is located in each of the one or the plurality of wells in the reaction vessel; the one or the plurality of wells are each covered with a sealant for preventing evaporation of the sample liquid such that the sealant is supported by the pillar; and the pillar prevents the sample liquid under measurement from being attached to the sealant for preventing evaporation of the sample liquid.
[14] A method for performing a PCR by use of the device according to any one of [1] through [13], the method comprising the steps of:
   (a) locating a sample liquid containing nucleic acid in a well;
   (b) switching a temperature of a reaction vessel from a nucleic acid elongation reaction temperature as a first temperature to (i) a nucleic acid denaturation temperature or (ii) the nucleic acid denaturation temperature or a nucleic acid annealing temperature as a second temperature;
   (c) continuously switching the temperature of the reaction vessel from the second temperature to the first temperature; and
   (d) repeating the step (b) and the step (c) a predetermined times at a predetermined time interval;
   wherein the switch of the temperature of the reaction vessel from the second temperature to the first temperature is performed by turning off an operation of a thermoelectric element.
[15] The method according to [14], wherein in the step (d), repeating the step (b)(ii) and the step (c) the predetermined times includes repeating, at a predetermined time interval, alternate performance of:
   switching the temperature of the reaction vessel from the nucleic acid elongation reaction temperature as the first temperature to (b)(ii) the nucleic acid denaturation temperature as the second temperature and continuously switching the temperature of the reaction vessel from the nucleic acid denaturation temperature to the first temperature; and
   switching the temperature of the reaction vessel from the nucleic acid elongation reaction temperature as the first temperature to (b)(ii) the nucleic acid annealing temperature as the second temperature and continuously switching the temperature of the reaction vessel from the nucleic acid annealing temperature to the first temperature.

Regarding the "additional mechanism that allows more stable temperature control", the reaction temperature controller according to the present invention includes means that realizes, for example, three temperatures used in a usual PCR reaction, namely, the denaturation temperature (hereinafter, referred to as the "high temperature"), the annealing temperature (hereinafter, referred to as the "low temperature"), and the elongation reaction temperature (hereinafter, referred to as the "middle temperature") in a short time. The reaction temperature controller especially provides, at high speed in a stably manner, the elongation reaction temperature, which needs to be adjusted, for example, extended, in accordance with the length of the DNA chain to be reacted. Namely, an embodiment of the present invention provides, for example, a high-speed gene amplification device including (1) a heat sink that has a large heat capacity and is controlled to be stable at the middle temperature; (2) a heating wire that supplies heat so as to keep the temperature of the heat sink at a certain level, or means that circulates, at high speed, a liquid of the middle temperature kept at a certain level; (3) a DNA reaction chip in which a sample to be subjected to the DNA elongation reaction is arranged; (4) a container which accommodates the DNA reaction chip and has an optically transparent window for wavelengths in a fluorescence observation range, which keeps the temperature of the top surface of the chip at 75°C or higher; (5) heat transfer means, such as a Peltier element or the like, that is arranged between the DNA reaction chip and the heat sink and can actively transfer the heat; and (6) means that detects a change in the fluorescence intensity of a liquid on the DNA reaction chip. In the case where a material having a large heat capacity and a heat source such as a heating wire or the like are used for the heat sink, it is desirable that the heat sink is formed of a metal material having a large heat capacity and a high heat conductivity (e.g., aluminum, duralumin, gold, silver, tungsten, Nepal brass, magnesium, molybdenum, beryllium, and an alloy containing such a metal element). As an equivalent to the heat sink, a heat exchange chamber using a high-speed reflux liquid that is set to have the middle temperature may be used. In this case, in a high-speed liquid reflux type reaction control device including only one reservoir or in a high-speed liquid reflux type reaction control device that includes two or three reservoirs and thus is usable for a two-temperature or three-temperature PCR, only one reservoir that is set to have the middle temperature may be used. For the DNA reaction chip in which the sample is located, the structure of the reaction container of the high-speed liquid reflux type PCR device (WO2010/113990) for which the present inventors filed a patent application in the past can be used with no modification.

### ADVANTAGEOUS EFFECTS OF INVENTION

The present invention for controlling the temperature of a reaction vessel by use of a heat sink or a heat exchange chamber set to have the middle temperature has advantages that 1) the temperature can be strictly controlled to the middle temperature, 2) the temperature can be stably kept at the middle temperature, and 3) overshoot can be avoided during the transition to the middle temperature. A reason why the problem of overshoot can be solved is that since the temperature of the heat sink or the heat exchange chamber having a large heat capacity is substantially kept at a certain level, the temperature of the surface of the reaction vessel and the temperature of the heat sink or the heat exchange chamber can be equilibrated almost instantaneously. According to the present invention, the heat capacities of the reaction vessel and the sample are insignificant as compared with that of the heat sink or the heat exchange chamber. In addition, the present invention has the following advantages regarding the operation of the thermoelectric element (e.g., Peltier element). The temperature of the heat sink or the heat exchange chamber is stabilized, and the temperature at the stabilization point (middle temperature) is in the middle between the high temperature and the low temperature. For these reasons, unlike in a conventional Peltier device-controlled gene amplification device that is operated from room temperature, the quantity of heat transfer during the transition to the high temperature or to the low temperature is minimized. In addition, as long as the temperature of the heat sink or the heat exchange chamber is stable, the quantity of electric current that is needed in order to change the temperature to the high temperature or the low temperature can be easily estimated. Even if the heat sink or the heat exchange chamber is locally deprived of heat by the operation of the Peltier element, no heat gradient is basically generated because the heat capacity of the heat sink or the heat exchange chamber is sufficiently large. Needless to say, when the thermoelectric element such as the Peltier element of the like is in an off state, the temperature of the reaction vessel does not exceed the temperature of the heat sink or the heat exchange chamber. According to the present invention, the temperature can be changed by 30°C or greater within 0.5 seconds. Hence, according to the present invention, the time required for changing the temperature can be made extremely short and, for example, the total time for completing a PCR can be made significantly shorter than the time required with a conventional device.

In order to perform a two-temperature or three-temperature PCR, the conventional liquid reflux type reaction control device requires a switching activation unit such as a valve or the like for alternately circulating liquids of different two or three temperatures from the liquid reservoirs to the heat exchange chamber. By contrast, the present invention provides an advantage that such a switching activation unit is not required. In the case where the present invention is carried out by use of a liquid reflux type reaction control device that includes a plurality of reservoirs and thus is usable for two-temperature or three-temperature settings, the present invention provides an advantage that the temperature can be easily set to a plurality of different middle temperatures by use of a switching mechanism such as a valve or the like.

### BRIEF DESCRIPTION OF DRAWINGS

[FIG. 1] FIG 1 is a schematic view showing an overall structure of a high-speed liquid reflux type reaction control device (e.g., PCR device).
[FIG. 2] FIG. 2 provides schematic views of a heat exchange chamber used in a high-speed liquid reflux type reaction control device.
[FIG. 3] FIG. 3 provides schematic views showing embodiments of a reaction vessel used in a device according to the present invention and methods for dissolving a lyophilized reagent.
[FIG. 4] FIG. 4 provides schematic views showing cylindrical reaction vessel casings used in a device according to the present invention and methods for attaching the cylindrical reaction vessel casings to the heat exchange chambers.
[FIG. 5] FIG. 5 provides schematic views showing a sequence of switching a valve used in a high-speed liquid reflux type reaction control device.
[FIG. 6] FIG. 6 provides diagrams showing (A) data regarding a temperature change and (B) results from a PCR, obtained by use of a high-speed liquid reflux type reaction control device.
[FIG. 7] FIG. 7 provides schematic views showing a glass-slide type reaction vessel casing used in a device according to the present invention and methods for attaching the glass-slide type reaction vessel casing to the heat exchange chamber.
[FIG. 8] FIG. 8 provides schematic views showing a driving mechanism for a slidable piston valve used in a high-speed liquid reflux type reaction control device.
[FIG. 9] FIG. 9 provides schematic views showing driving mechanisms for a slidable piston valve used in a high-speed liquid reflux type reaction control device.
[FIG. 10] FIG. 10 provides schematic views showing a driving mechanism for a rotary valve used in a high-speed liquid reflux type reaction control device.
[FIG. 11] FIG. 11 provides schematic views showing a temperature change mechanism with a membrane used in a high-speed liquid reflux type reaction control device.
[FIG. 12] FIG. 12 provides schematic views showing a driving mechanism for a temperature-setting valve used in a high-speed liquid reflux type reaction control device.
[FIG. 13] FIG. 13 is a schematic view presenting an example of position of a DNA reaction chip, temperature control on a top surface thereof, and a method for detecting fluorescence, which are shown by use of an example of structure of a high-speed liquid reflux type reaction control device.
[FIG. 14] FIG. 14 is a schematic view presenting an example of position of a DNA reaction chip, temperature control on a top surface thereof, and a method for detecting fluorescence, which are shown by use of an example of structure of a reaction vessel of a high-speed liquid reflux type reaction control device.
[FIG. 15] FIG. 15 provides schematic views presenting an example of position of a DNA reaction chip, temperature control on a top surface thereof, and a method for detecting fluorescence, which are shown by use of an example of structure of a transportation system for a reaction vessel provided in the form of a reaction vessel chip and of a device that performs a reverse transcription reaction of a high-speed liquid reflux type reaction control device.
[FIG. 16] FIG. 16 provides schematic views showing an example of method for detecting fluorescence of a sample by use of a reaction vessel according to the present invention.
[FIG. 17] FIG. 17 is a schematic view showing an example of method for detecting fluorescence of a sample by use of a reaction vessel according to the present invention.
[FIG. 18] FIG. 18 provides schematic views showing an example of structure of a reaction vessel according to the present invention.
[FIG. 19] FIG. 19 provides schematic views showing an example of structure of a reaction vessel and an example of detection method according to the present invention.
[FIG. 20] FIG. 20 provides schematic views showing an example of structure of a reaction vessel according to the present invention.
[FIG. 21] FIG. 21 is a schematic view showing an example of structure of a high-speed liquid reflux type reaction control device.
[FIG. 22] FIG. 22 is a schematic view showing an example of structure of an optical heating type reaction control device.
[FIG. 23] FIG. 23 provides schematic views showing the angle dependence of the transmittance of an ND filter in an example of structure of an optical heating type reaction control device.
[FIG. 24] FIG. 24 is a schematic view showing an example of structure of the present invention.
[FIG. 25] FIG. 25 provides graphs schematically showing a temperature change of a DNA sample and an operation of a Peltier element according to the present invention.

### DESCRIPTION OF EMBODIMENTS

Hereinafter, embodiments of the present invention will be described with reference to the drawings although these embodiments are provided for illustration only and do not limit the scope of the present invention.

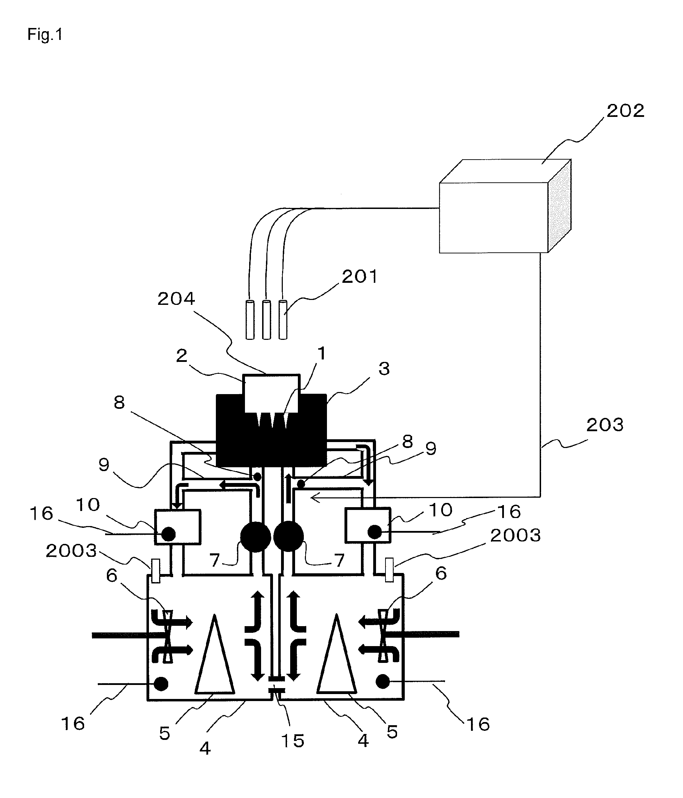

FIG. 1 is a schematic view showing an overall structure of one embodiment of a high-speed liquid reflux type reaction control device. Typically, this reaction control device includes a reaction vessel 1, a reaction vessel casing 2, a heat exchange chamber 3, liquid reservoir tanks 4, heat sources 5, stirring mechanisms 6, pumps 7, switching valves 8, bypass flow channels 9, and auxiliary temperature control mechanisms 10. Preferably, the reaction control device according to the present invention further includes fluorescence detectors 201, a control analyzer 202 that transmits a control signal 203, and an optical window (or aperture) 204.

In a preferable embodiment, the plurality of liquid reservoir tanks 4 are connected to each other by a coupling tube 15 in which a minute amount of liquid can be transferred between the tanks so as to prevent a difference in the liquid surface level from occurring between the tanks while the liquid is circulating at high speed, and thus to prevent a difference in the pressure from occurring between the tanks. Referring to FIG. 1, in order to allow heat exchange to be performed within a short period of time, the liquid is constantly circulated from each reservoir tank 4 by the pump 7. Even when the liquid is not to be led to the heat exchange chamber 3, the liquid is led to the bypass flow channel 9 by the switching valve 8 so that the liquid is constantly circulated. The circulating liquid is controlled to reflux such that the temperature thereof is fine-tuned to the temperature of the liquid in the corresponding reservoir tank 4 by the auxiliary temperature control mechanism 10 before the liquid returns to the reservoir tank 4. The auxiliary temperature control mechanisms 10 may each include a warming mechanism and a cooling mechanism so as to be capable of both warming and cooling the liquid by use of a Peltier element or the like. Alternatively, the auxiliary temperature control mechanisms 10 may each include a warming system by use of a resistive heating mechanism and an air-cooling fin type cooling mechanism. A liquid temperature sensor 16 is located in each reservoir tank 4 and also in each auxiliary temperature control mechanism 10. The heat sources 5 and the auxiliary temperature control mechanisms 10 can be controlled such that the liquid temperature can be adjusted to a desired level based on temperature information from the sensors. Desirably, the liquid temperature sensors may each be a thermistor formed of a material that is not corroded even when being in direct contact with the liquid, an anticorrosion covered thermocouple or the like.

In a preferable embodiment, a pressure leak valve 2003 is located at each reservoir tank. In the case where the pressure in the reservoir tank is increased due to gas such as water vapor or the like that is generated by the heat supplied from the heat source, the pressure leak valve 2003 effectively discharges the generated gas from the tank in order to prevent the tank from being destroyed and also in order to prevent the liquid surface level in the tank from becoming different from that in the other tank via the coupling tube due to the gas pressure difference.

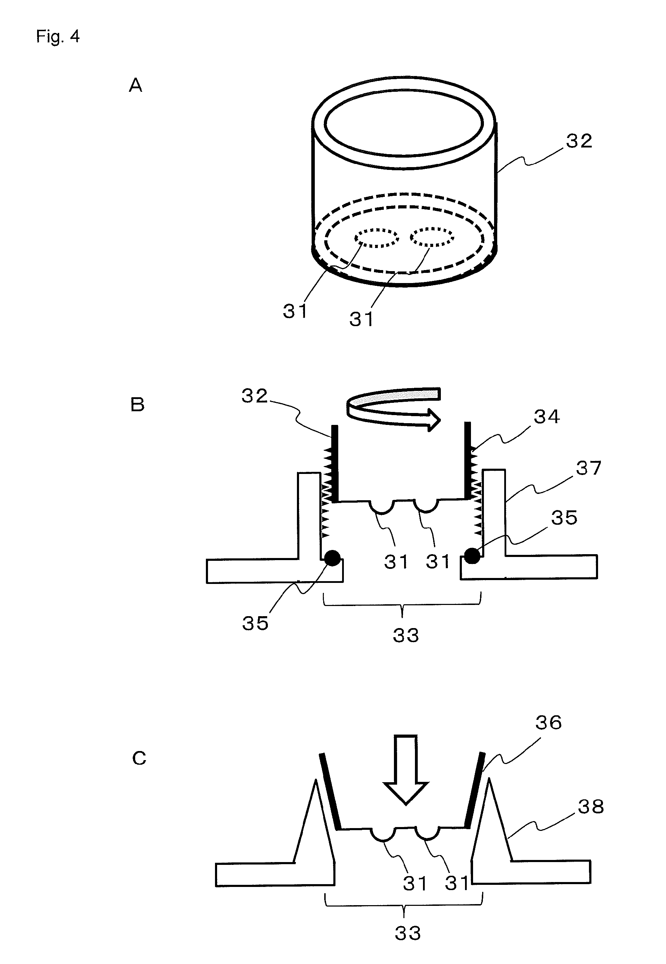

The reaction vessel 1 is typically formed of, for example, an aluminum, nickel or gold thin plate having a plurality of wells. Preferably, the thin plate has a smaller thickness in well regions than in the surrounding area so that the well regions have higher heat conductivity. The thickness of the well regions is typically, but not limited to, about 10 to 30 microns. The area between adjacent wells is preferably thicker in order to guarantee the overall strength, and the thickness of this area is typically in the range of, but not limited to, 100 microns to 500 microns. The reaction vessel 1 is typically secured to a bottom surface of the reaction vessel casing 2 to be formed integrally therewith. The bottom surface of the reaction vessel casing 2 is, for example, quadrangular or circular. Typically, the reaction vessel 1 and the reaction vessel casing 2 are detachable from the heat exchange chamber 3 (see FIG. 4).

The temperature of the liquid to be introduced into the heat exchange chamber 3 is controlled by each heat source 5 disposed inside each liquid reservoir tank 4. Preferably, the stirring mechanism 6 is provided in order to rapidly conduct the heat away from a surface of the heat source 5 and thus even out the temperature inside the liquid reservoir tank 4. The liquid in each liquid reservoir tank 4 is led to the inside of the flow channel by the pump 7. The liquid is switched by the switching valve 8 to be led to the heat exchange chamber 3 or to directly return to the liquid reservoir tank 4 through the bypass flow channel 9 without being led to the heat exchange chamber 3. If necessary, each auxiliary temperature control mechanism 10 performs delicate control such that the temperature of the liquid which has been changed during the circulation is corrected to the level set for the tank 4 before the liquid is discharged. Thus, temperature fluctuation inside the liquid reservoir tank 4 is suppressed.

The liquid to be introduced into the heat exchange chamber 3 may be, but not limited to, water, and may be any liquid which has a large heat capacity and a low viscosity (e.g., liquid ammonia). It should be noted that a nontoxic and nonflammable liquid is desirable from the viewpoint of safety. For example, a liquid having a higher boiling point than that of water may be used to ensure that the temperature of a sample solution is 100°C, or a liquid having a lower freezing point than that of water may be used to ensure that the temperature is changed down to the freezing point of water while preventing solidification of the liquid circulating within the device.

Preferably, as shown in FIG. 1, the reaction vessel casing 2 includes the optical window 204 that allows transmission of fluorescent dye-exciting light and fluorescence, such that change in the fluorescence intensity of the fluorescent dye in a sample solution that occurs in accordance with the reaction of the sample solution in the reaction vessel 1 can be measured for one or each of the plurality of reaction vessels. The fluorescence detectors 201 each measures time-wise change in the fluorescence intensity in the corresponding reaction vessel 1. In the example shown in FIG. 1, the plurality of fluorescence detectors 201 each include an exciting light irradiation mechanism and a fluorescence detecting mechanism. When, for example, a PCR is to be conducted, this structure allows independent measurement of PCR amplification information on each of the plurality of reaction vessels 1 containing different primers or different sample solutions. In addition, data on the fluorescence intensity acquired by each fluorescence detector 201 is recorded by the control analyzer 202, which has a function of estimating the amount of DNA or mRNA in the sample solution obtained by the PCR. The control analyzer 202 also has a function of acquiring switching information on each switching valve 8 and thus estimating whether or not the temperature of the sample solution after valve switching has reached the target temperature based on the time-wise change in the fluorescence intensity, and also has a mechanism of controlling the valve switching based on the result. The above-described estimation is performed by utilizing that fluorescence quenching based on the mobility of water molecules that are universally possessed by a fluorescent dye depends on the liquid temperature, and is performed based on a decrease or nulling in the amount of change of the fluorescence intensity per unit time. This is particularly effective for confirming that a high temperature state which results in DNA denaturation has been achieved.

In the example shown in FIG. 1, one detector is provided for each reaction vessel 1. Alternatively, a fluorescence-exciting light source may be combined with a camera capable of quantitating and detecting fluorescence such as a cooled CCD camera or the like to measure the change in the fluorescence intensity of the plurality of reaction vessels 1. Still alternatively, when the number of detectors used is less than the number of the reaction vessels 1, a mechanical driving mechanism capable of travelling on an X-Y plane at high speed may be combined with the detectors to measure the fluorescence intensities of all of the reaction vessels 1.

The volume of the sample solution can be in the range of, but not limited to, 0.1 µL to 100 µL per well. A preferable volume of the sample solution is 0.1 to several ten (e.g., 90, 80, 70, 60, 50, 40, 30, 20 or 10) microliters per well. When necessary, a smaller volume of, for example, 0.5 µL to 10 µL per well, 1 µL to 5 µL per well, 1 µL to 2 µL per well or the like is also preferable. The wells may contain, in addition to the sample solution, mineral oil or the like that prevents evaporation of the sample solution. The volume of the mineral oil is preferably, but not limited to, about several microliters (e.g., 3 to 4 µL), and is appropriately changeable in accordance with the size of the well or the amount of the sample as obvious to a person of ordinary skill in the art.

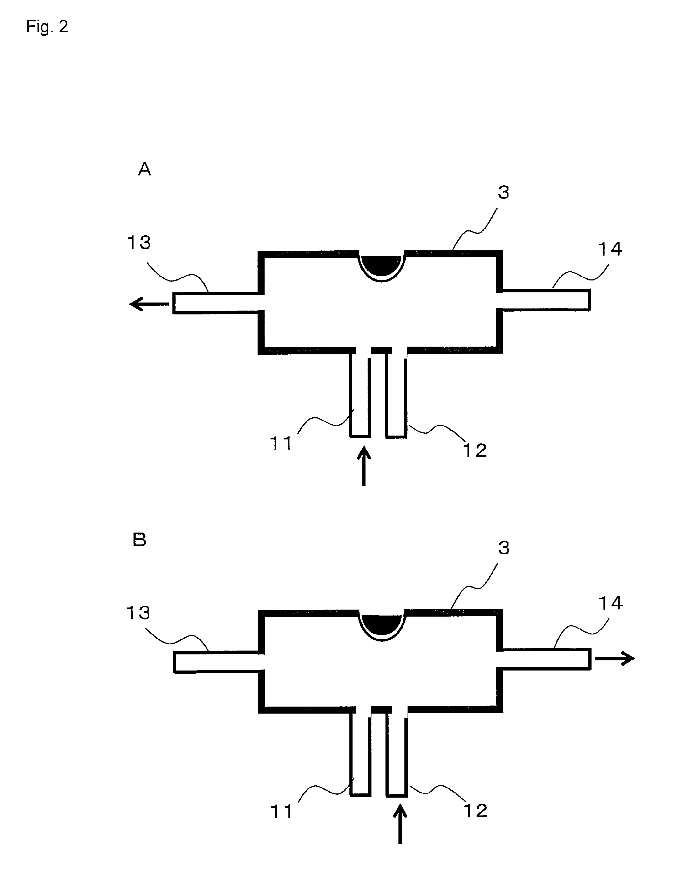

FIG. 2 provides schematic views of the heat exchange chamber 3 used in the high-speed liquid reflux type reaction control device. Basically, the heat exchange chamber 3 includes inlets A (11) and B (12) for introducing liquids of different temperatures. The heat exchange chamber 3 also includes a plurality of outlets, i.e., outlets A (13) and B (14), for returning the liquid in the heat exchange chamber 3 to the liquid reservoir tanks 4. FIG. 2A schematically shows that a liquid of a certain temperature is introduced from one of the liquid reservoir tanks 4 via the inlet A (11) and is discharged via the outlet A (13). FIG. 2B schematically shows that a liquid of a different temperature is introduced from the other liquid reservoir tank 4 via the inlet B (12) and is discharged via the outlet B (14). The number of the inlets is not limited to two, and the inlets may be provided in any number that matches the number of levels to which the temperature of the sample solution is to be changed. Namely, the inlets may be provided for two or more temperatures. For example, in order to realize a three-temperature system, the number of the inlets is three. Similar to the case of the inlets, the number of the outlets is not limited to two, either. The arrows in FIG. 2 roughly indicate the flowing directions of the liquids introduced into, or discharged from, the heat exchange chamber 3.

The total volume of the liquids to be circulated between the heat exchange chamber 3 and the liquid reservoir tanks 4 is as follows, considering the flow rate, the heat capacity and the temperature stability of the liquids to be circulated. In the case where the flow rate is 10 mL per second or larger in order to realize high-speed temperature change and the temperature stability of the reaction vessel 3, the total volume of the liquids is usually several ten milliliters or larger, preferably 100 mL or larger, more preferably 200 mL or larger, and most preferably 300 mL or larger. The upper limit of the volume may appropriately be determined in consideration of the portability of the device or the like.

The capacity of the heat exchange chamber 3 is preferably at least about 10 times, more preferably at least about 100 times, and most preferably at least about 1000 times the amount of the sample per well. Typically, the capacity of the heat exchange chamber is about 0.01 mL to 10 mL per well, more preferably about 0.05 mL to several milliliters (e.g., 9, 8, 7, 6, 5, 4, 3, 2 or 1 mL) per well, and most preferably about 0.1 mL to 2 mL per well.

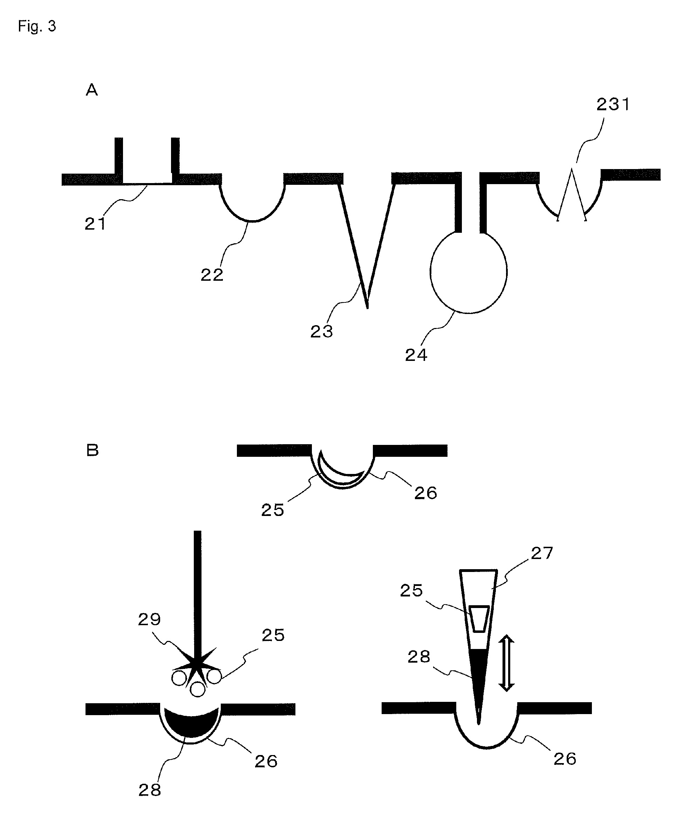

FIG. 3 provides schematic views showing embodiments of the reaction vessel usable in the device according to the present invention and methods for dissolving a lyophilized reagent according to the present invention. The reaction vessels or wells may be provided in any of various shapes. FIG. 3A shows, as examples, a reaction vessel A (21) in which a surface contacting the liquid in the heat exchange chamber is flat, a reaction vessel B (22) in which the surface is hemispherical, a reaction vessel C (23) in which the surface is trigonal pyramid-shaped, a reaction vessel D (24) in which the surface is spherical, and a reaction vessel C' (231) in which the surface is spherical but has a conical structure projecting into the spherical recess. This reaction vessel C' can stably maintain the position of the liquid droplets and increase the area of the surface contacting the liquid. For providing higher efficiency of heat conduction, it is preferable that the area of the surface contacting the liquid in the heat exchange chamber is as large as possible as readily understood by a person of ordinary skill in the art.

It is convenient that the reagent necessary for the reaction is lyophilized. Referring to FIG. 3B, a lyophilized reagent 25 can be prepared and placed at the bottom of a reaction vessel 26. Alternatively, a plug-shaped lyophilized reagent 25 may be provided inside a dispensing chip 27 used for dispensing the sample so that the reagent is dissolved in a sample solution 28 by shaking the sample solution 28 up and down. Still alternatively, a lyophilized reagent 25 may be provided on a surface of a fiber ball 29 made of a bundle of nylon fibers or the like so that the lyophilized reagent is dissolved by inserting and stirring the fiber ball in a sample 28 in the reaction vessel 26.

FIG. 4 provides schematic views showing a cylindrical reaction vessel casing 32 usable in the device according to the present invention and methods for attaching the cylindrical reaction vessel casing 32 to a heat exchange chamber 37 according to the present invention. Directly handling a reaction vessel formed of a thin membrane is inconvenient. It is convenient that a reaction vessel 31 is secured to the reaction vessel casing 32 as shown in FIG. 4A. The reaction vessel casing 32 is desirably formed of a heat insulating material such as polystyrene, polycarbonate, PEEK, acrylic resin or the like. An area of the reaction vessel casing 32 that is joined to the reaction vessel 31 is desirably minimized (e.g., to 5 mm² or smaller) for rapid and highly precise temperature increase and decrease of the reaction vessel 31.

FIG. 4B shows, as one embodiment of attaching the reaction vessel 31 to the heat exchange chamber 37, a method by which a thread 34 is formed in a surface of the reaction vessel casing 32 and the reaction vessel casing 32 is screwed into a reaction vessel socket 33 of the heat exchange chamber 37. As shown in FIG. 4B, the opening is desirably provided with a seal 35 in order to maintain water tightness. FIG. 4C shows another method. Referring to FIG. 4C, a tapered reaction vessel casing 36 may be employed so as to attach the reaction vessel 31 to a heat exchange chamber 38 by pressure only.

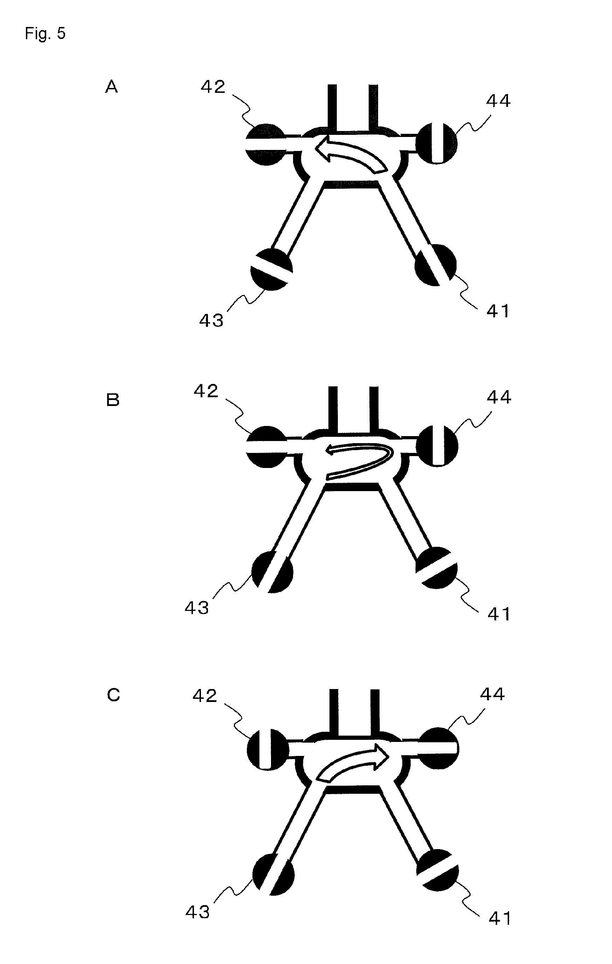

FIG. 5 shows specific examples of valve switching mechanism used in the high-speed liquid reflux type reaction control device. FIG. 5 shows inlet valves A (41) and B (43) for introducing a liquid into a reaction vessel and outlet valves A (42) and B (44) for leading the liquid outside. The liquid led in via the inlet valve A (41) returns to one of the liquid reservoir tanks 4 via the outlet valve A (42), whereas the liquid led in via the inlet valve A (43) returns to the other liquid reservoir tank 4 via the outlet valve B (44). By alternately switching these two states, the sample in the reaction vessel can be brought into reaction. According to a more preferable valve switching method, a state is generated where the inlet valve B (43) and the outlet valve A (42) or the inlet valve A (41) and the outlet valve B (44) are opened at the same time for a moment, in addition to the above-described two states. In this manner, liquids of different temperatures can be suppressed from mixing with each other, which facilitates the temperature control on the liquid reservoir tank in each system.

The circulating rate of the liquid is not particularly limited, but is generally about 1 mL/sec. to 100 mL/sec., more preferably 5 mL/sec. to 50 mL/sec., and most preferably 7 mL/sec. to 15 mL/sec. In order to circulate the liquid in each reservoir tank to the heat exchange tank 3 without decreasing the temperature of the liquid, it is desirable that the liquid is constantly circulated at high speed of 10 mL/sec. or higher from the reservoir tank by the pump 7.

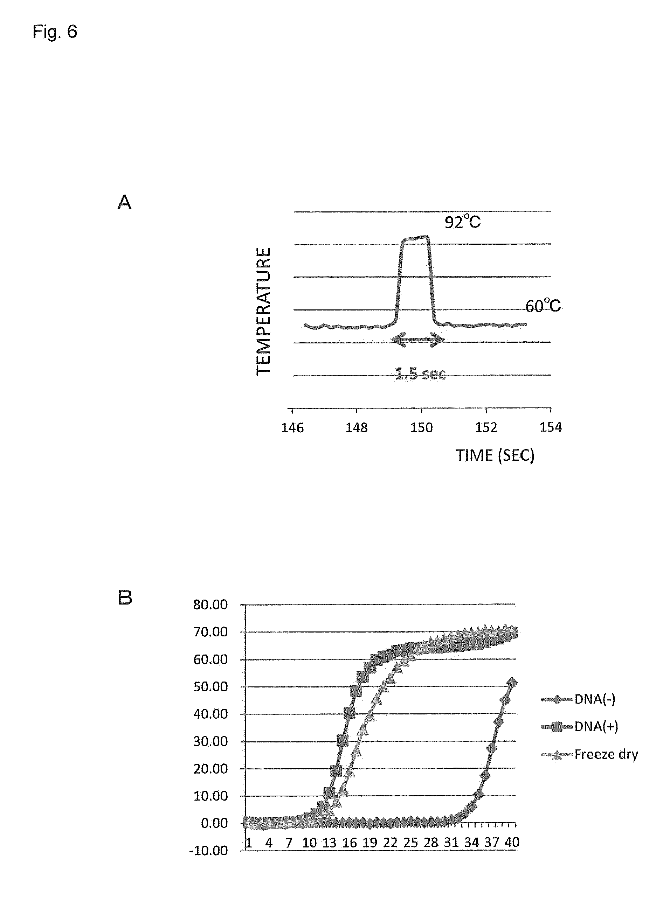

FIG. 6A is a graph obtained from data on temperature control realized by using the above-described mechanism. As shown in FIG. 6A, the temperature can be increased from 60°C to 92°C and decreased back to 60°C within a short time of 1.5 seconds. FIG. 6B is a graph showing the results from a real-time PCR. The conditions of the solution for carrying out the PCR were as follows. The followings were mixed in the following proportion: 1.0 µL of reaction buffer, 1 µL of 2 mM dNTP (dATP, dCTP, dGTP, dTTP), 1.2 µL of 25 mM magnesium sulfate, 0.125 µL of 10% fetal bovine serum, 0.5 µL of SYBR Green I, 0.6 µL each of two types of primers, 3.725 µL of sterile water, 0.25 µL of KOD plus polymerase, and 1.0 µL of genomic DNA. The temperature was 95°C for the first 10 seconds, and then the cycle of maintaining the temperature at 95°C for 1 second, changing the temperature to 60°C and maintaining the temperature at 60°C for 3 seconds were repeated 40 times. The circulating rate of the liquid was about 10 mL/sec.

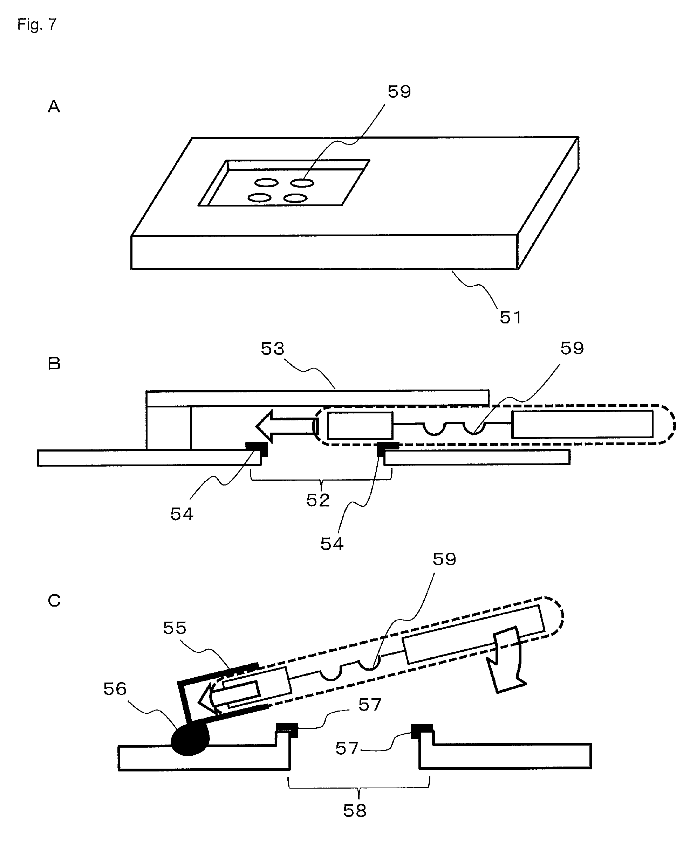

FIG. 7 shows variations of a method for attaching or detaching a reaction vessel 59 and a reaction vessel casing 51 usable in the device according to the present invention to or from the heat exchange chamber. The reaction vessel 59 in an extended state is attached to the glass-slide type reaction vessel casing 51 (FIG. 7A). In order to attach the glass-slide type reaction vessel casing 51 to the heat exchange chamber, the reaction vessel casing 51 may be slid along a guide rail 53 and pressed against a seal 54 to be fixed (FIG. 7B). Alternatively, the glass-slide type reaction vessel casing 51 may be inserted into a slide socket 55 and pressed against a seal 57 utilizing a hinge 56 (FIG. 7C).

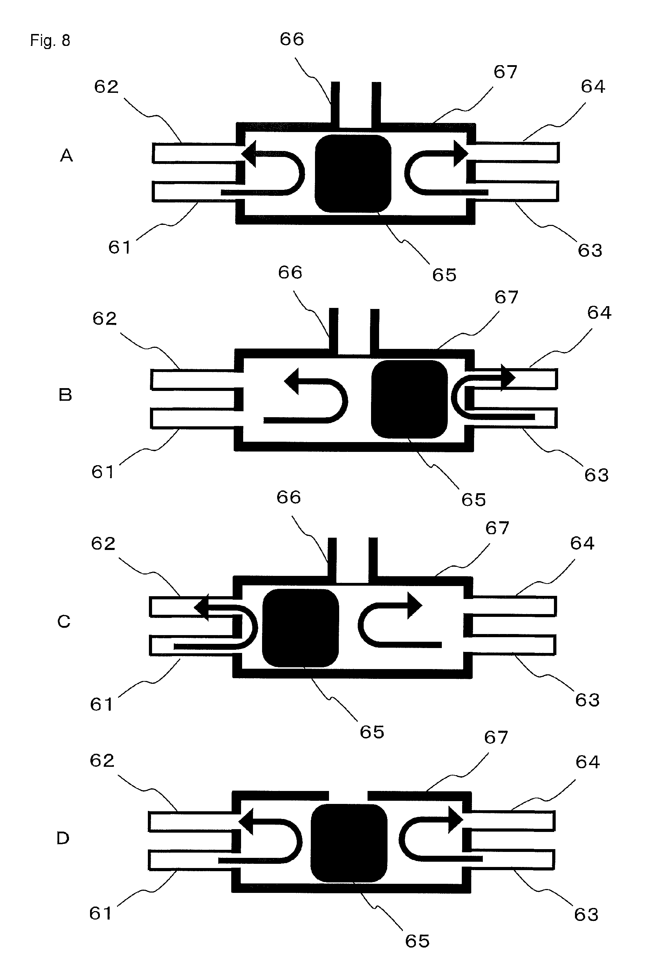

FIG. 8 provides schematic views showing variations of a valve switching mechanism used in the high-speed liquid reflux type reaction control device, and shows driving mechanisms for a slidable piston valve, which is different from the valve shown in FIG. 5. A piston 65 that is slidable leftward and rightward is used as a valve mechanism that changes the temperature of a reaction vessel 66. On the left side of the piston 65, a liquid is introduced into a heat exchange chamber 67 via an inlet A (61) and led outside via an outlet A (62). On the right side of the piston 65, a liquid is introduced into the heat exchange chamber 67 via an inlet B (63) and led outside via an outlet B (64). When the piston 65 slides rightward with respect to the reaction vessel 66, the temperature of the reaction vessel 66 comes to equilibrium with that of the liquid introduced via the inlet A (61). By contrast, when the piston 65 slides leftward, the temperature of the reaction vessel 66 comes to equilibrium with that of the liquid introduced via the inlet B (63). When the piston 65 is positioned just below the reaction vessel 66, the reaction vessel 66 can be detached without leakage of the liquid. Desirably, the piston 65 is formed of a material having high heat insulation property, or is hollow and the inner space is filled with gas or is in a vacuum state. The arrows in FIG. 8 roughly indicate the flowing directions of the liquids.

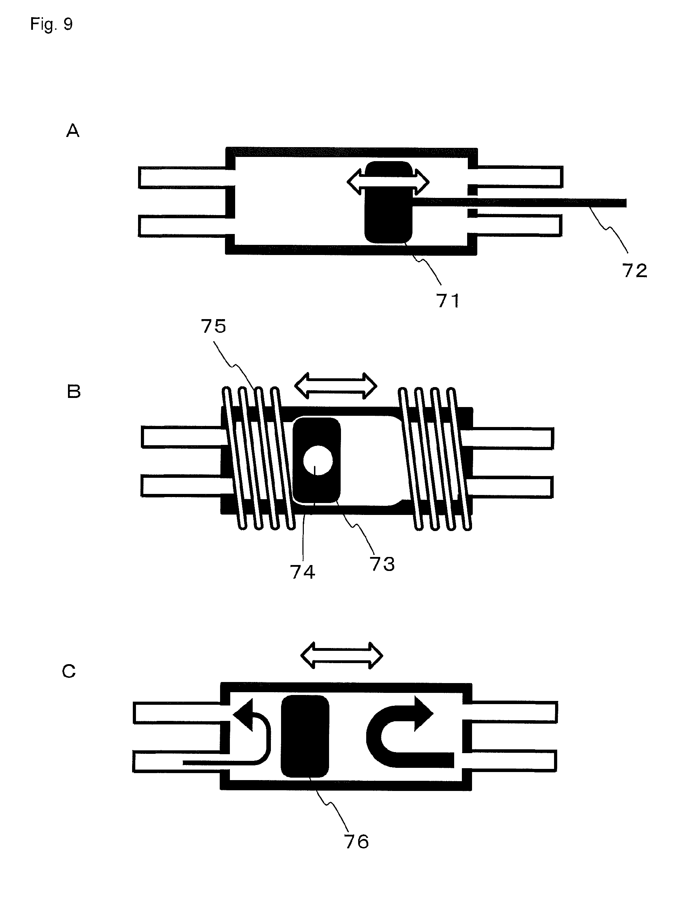

FIG. 9 shows some variations of a driving mechanism for a piston of a piston valve used in the high-speed liquid reflux type reaction control device. According to one method, a piston 71 is integrally formed with a piston rod 72 and is directly operated from outside (FIG. 9A). According to another method, a piston 73 is formed of a ferromagnetic material such as iron, nickel or the like, or a magnet 74 is put inside the piston made of any other material. An electromagnetic coil 75 is externally provided to control the current to slide the piston 73 leftward and rightward (FIG. 9B). According to still another method, the pressure on the inlet side or the fluid resistance at the outlet is controlled to slide a piston 76 leftward and rightward by utilizing the difference in the pressure between the two sides of the piston 76 (FIG. 9C). In FIG. 9, the white arrows indicate the directions of the movement of the piston, whereas the black arrows indicate the flowing directions of the fluids. The orientation of each arrow roughly shows the flowing direction, and the width of each arrow roughly shows the flow rate of the fluid.

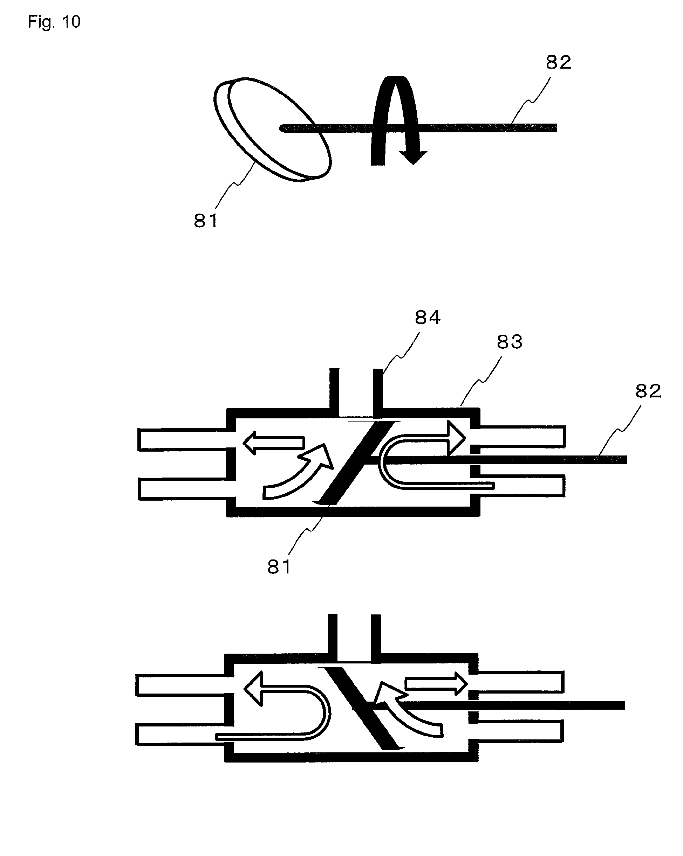

FIG. 10 shows another embodiment of a valve switching mechanism used in the high-speed liquid reflux type reaction control device. A rotary valve 81 formed of a slanted oval plate attached to a rod 82 as a rotation shaft is inserted into a heat exchange chamber 83 having a circular cross-section. The rotary valve 81 divides the heat exchange chamber 83 into a right part and a left part, and rotation of the rotation shaft 82 can lead a liquid introduced from the right side or the left side of the heat exchange chamber to a reaction vessel 84. The rotary valve 81 is a slanted flat plate in FIG. 10, but may have any other shape such as the shape of a spiral screw or the like. The rotary valve 81 may have any shape as long as a similar effect is provided by rotating the rotation shaft. In FIG. 10, the black arrow in FIG. 10 indicates the rotation direction of the rotation shaft 82, whereas the white arrows roughly indicate the flows of the liquids.

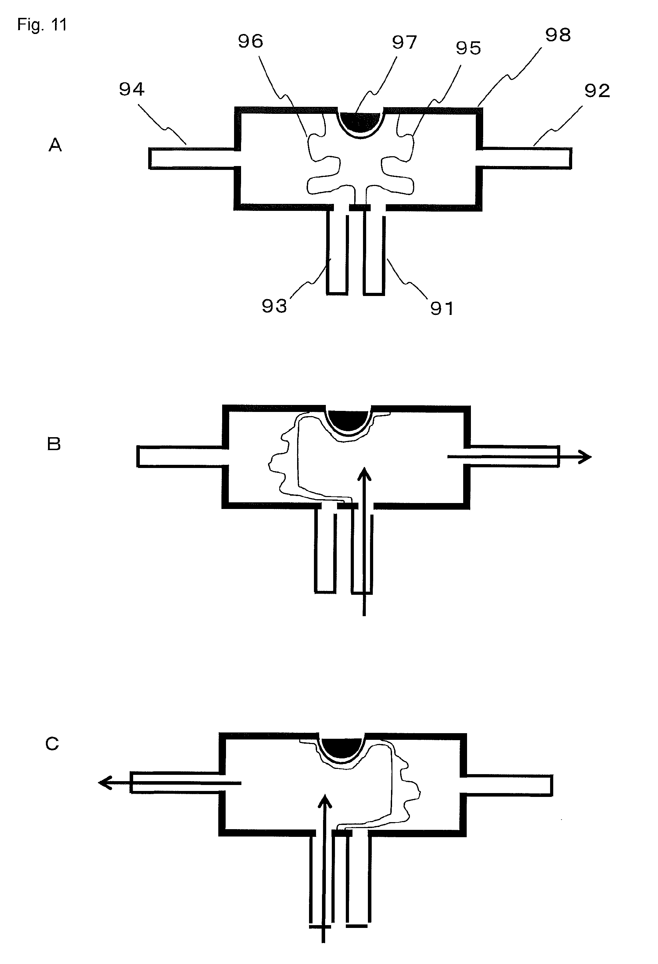

FIG. 11 shows a structure of replacing a liquid by an element other than a valve. A heat exchange chamber 98 is divided by a membrane A (95) and a membrane B (96). A liquid introduced via an inlet A (91) is led outside via an outlet A (92). The presence of the membranes prevents the liquid from being led outside via an inlet B (93) or an outlet B (94) (FIG. 11 A). When the pressure of the liquid introduced via the inlet A (91) is higher than the pressure of a liquid introduced via the inlet B (93), the membranes A (95) and B (96) are pushed leftward so that the heat of the liquid introduced via the inlet A (91) is conducted to a reaction vessel 97 (FIG. 11B). When the pressure relationship between the liquid introduced via the inlet A (91) and the liquid introduced via the inlet B (93) is reversed, the temperature of the reaction vessel 97 comes to equilibrium with the temperature of the liquid introduced via the inlet B (93) (FIG. 11C). The membranes are desirably formed of a thin film of heat-resistant rubber or the like which has a high heat resistance. The arrows shown in FIG. 11 roughly indicate the flowing directions of the liquids.

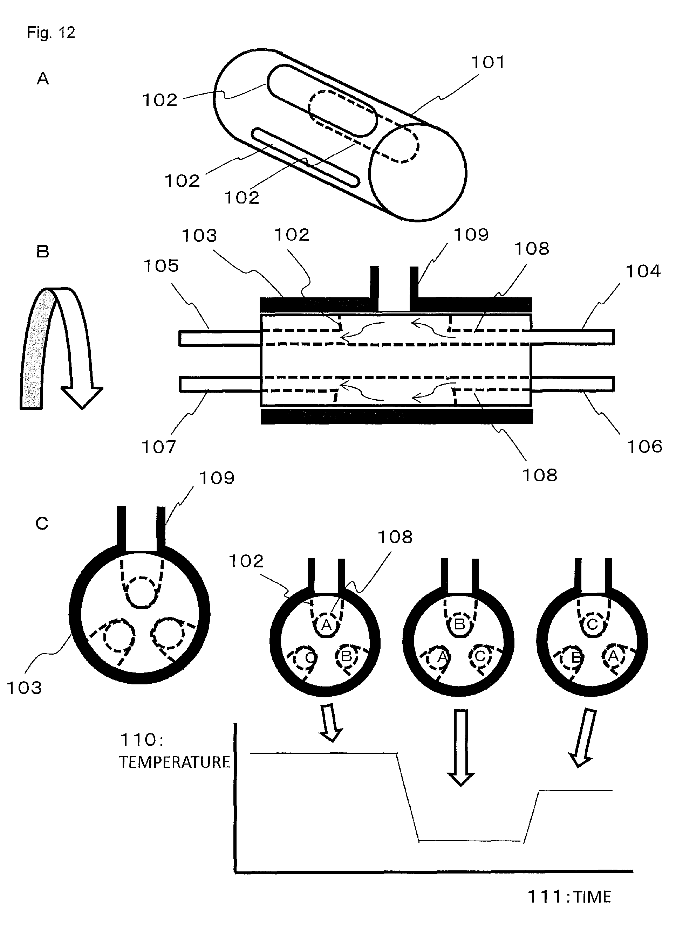

FIG. 12 provides schematic views showing still another driving mechanism for a temperature-setting valve used in the high-speed liquid reflux type reaction control device. In this mechanism, the number of temperatures to be set is not limited to two. FIG. 12 shows a structure by which three or more temperatures can be set for the reaction vessel. A rotary valve 101 having grooves 102 formed in a circumferential surface thereof is inserted into a heat exchange chamber 103. The rotary valve 101 includes an inlet and an outlet respectively on two sides thereof. For example, a liquid introduced via an inlet A (104) flows into the groove 102 via a flow channel 108 to conduct heat to a reaction vessel 109 and then are led outside via an outlet A (105). By contrast, a liquid introduced via an inlet B (106) is led outside via an outlet B (107) without making contact with the reaction vessel 109. However, the rotary valve 101 may be rotated such that the liquid introduced via an arbitrary inlet can be brought into contact with the reaction vessel (FIG. 12C). The rotary valve 101 may be rotated along elapsed time 111 so that temperature 110 can be changed as shown in the graph in FIG. 12C. The rotary valve 101 is desirably formed of a heat insulating material.

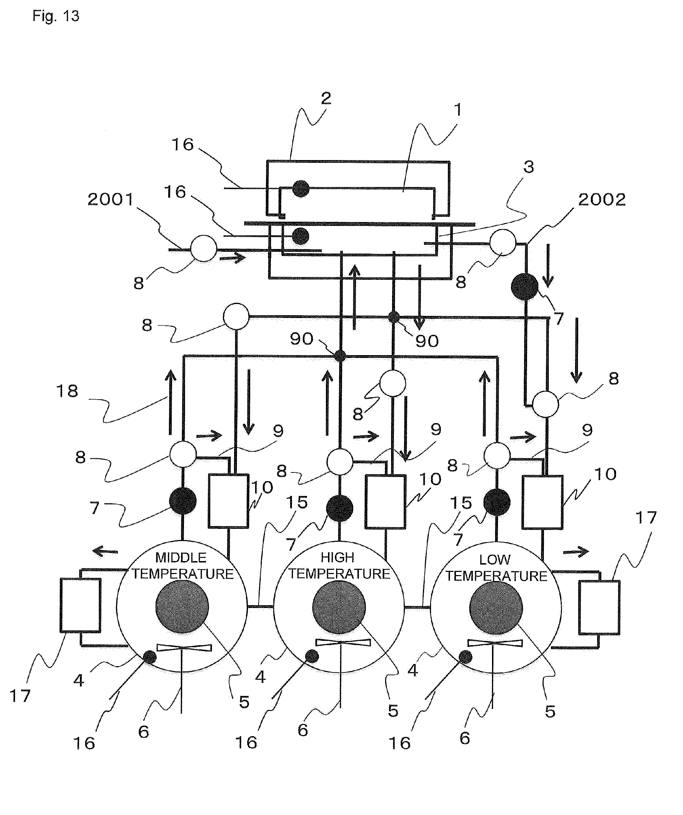

FIG. 13 is a schematic view showing an overall structure of an embodiment of a temperature control mechanism. In FIG. 13, a control part of a three-temperature system of a high-speed liquid reflux type reaction control device is omitted. The reaction control device for causing a high-speed PCR typically includes a reaction vessel 1 used to perform heat exchange between a liquid circulating at high speed and PCR liquid droplets, a reaction vessel casing 2 that prevents the PCR liquid droplets on the reaction vessel from being contaminated with an external substance and also prevents evaporation of the liquid droplets, a heat exchange chamber 3 into which a high-speed circulating liquid for heat exchange is introduced, three liquid reservoir tanks 4 that hold liquids at three different temperatures respectively. The reaction control device according to the present invention also includes a heat source 5, a stirring mechanism 6, a pump 7, a switching valve 8, a bypass flow channel 9, and an auxiliary temperature control mechanism 10 which are provided for each reservoir tank. In order to realize a high-speed PCR, the liquid from each reservoir tank circulates in the liquid flowing directions represented by arrows 18. The flows from the reservoir tanks are joined together at a joint 90 via the switching valves 8, and are led to the flow channel toward the heat exchange chamber 3. The liquid flowing out from the heat exchange chamber 3 is branched, at a joint 90 provided on a downstream side, into reflux flow channels leading to the reservoir tanks. The switching valves 8, located on the branch flow channels on both of the upstream side and the downstream side and provided respectively for high-temperature, middle-temperature and low-temperature liquids circulating at high speed, are switched at the same time in association with each other to change the liquid to be introduced into the heat exchange chamber 3 to any one of the high-temperature liquid, the middle-temperature liquid and the low-temperature liquid instantaneously. FIG. 13, which is a schematic view provided for the purpose of showing the structure of the device and the locations of the tubes in an easy-to-see manner, does not reflect the actual lengths of the tubes between the elements in the device. It is preferable that the switching valves 8 are each connected to the corresponding joint 90 by a tube having such a length that almost no amount of high-speed circulating liquid stays in the tube; namely, it is preferable that the switching valves 8 are each located just adjacent to the corresponding joint 90. In order to minimize the temperature drop of the high-speed circulating liquid supplied from each reservoir tank 4 to the heat exchange chamber 3, it is preferable that a minimum possible number of parts, for example, two parts (mechanisms), i.e., the pump 7 and the switching valve 8, are located between each reservoir tank 4 and the heat exchange chamber 3 and thus the length of the flow channel from the reservoir tank 4 to the heat exchange chamber 3 is minimized. By contrast, the length of the flow channel through which the liquid refluxes from the heat exchange chamber 3 to each reservoir tank 4 may be slightly longer with no problem because the temperature of each liquid is corrected by the auxiliary temperature control mechanism 10 before the liquid refluxes to the reservoir tank 4. The pump 7 is located between each reservoir tank 4 and the switching valve 8 on the upstream side that supplies the liquid to the heat exchange chamber 4, so that the high-speed refluxing liquid is constantly "pushed" directly from the reservoir tank 4. The pump 7 is located at such a position in order to supply the liquid to the heat exchange chamber 3 at high speed with certainty, and also in order to, when the liquid is not supplied to the heat exchange chamber 3, reflux the liquid to each reservoir tank 4 by the switching valve via the auxiliary temperature control mechanism 10 instantaneously and with certainty. In order to minimize the temperature drop of the high-speed circulating liquids supplied from the reservoir tanks 4 to the heat exchange chamber 3, the flow channels preferably have an inner diameter of 2 mm or greater.

As described above in the example shown in FIG. 1, a liquid temperature sensor 16 that measures the temperature of the liquid is located in each reservoir tank and each auxiliary temperature control mechanism. Based on information from each sensor 16, the temperature of the reservoir tank 4 realized by the heat source 5 can be controlled, and the temperature of the liquid passing the auxiliary temperature control mechanism 10 can be fine-tuned to the temperature of the reservoir tank 4. Since the auxiliary temperature control mechanism 10 is provided, the reservoir tank 4 merely needs to have a minimum level of temperature buffering function. This can minimize the capacity of the reservoir tank 4, namely, the amount of the circulating liquid. In this example, a three-temperature PCR corresponding to three states of denaturation, annealing and elongation can be performed as opposed to the two-temperature PCR in the example shown in FIG. 1. Now, control on the three temperatures, namely, a high temperature, a middle temperature and a low temperature will be described. Regarding the reservoir tank for the high temperature (in FIG. 13, the reservoir tank 4 in the middle), the temperature can be sufficiently controlled merely by a warming system because the temperature is maintained at 95°C or higher because of denaturation. However, regarding the reservoir tanks for the middle and low temperatures, the reaction temperatures, namely, the temperatures of the reservoir tanks may occasionally need to be fine-tuned in accordance with the type of enzyme used. The temperature of the liquid in the each reservoir tank 4 can be easily increased by supplying heat from the heat source. The temperature of the liquid in the each reservoir tank 4 can be decreased by, for example, an auxiliary liquid heat release mechanism 17 attached to each of the two reservoir tanks 4. The auxiliary liquid heat release mechanism 17 is operated only when the temperature of the liquid in the reservoir tank 4 needs to be decreased. The liquid in the reservoir tank is absorbed at high speed by a pump system included in the mechanism, and the heat of the liquid is released in a process in which the liquid flows in the flow channel provided with a cooling mechanism such as an air-cooling type cooling mechanism, a Peltier element or the like. The liquid is cooled until it is confirmed by the liquid temperature sensor 16 located in the reservoir tank 4 that the liquid temperature has been decreased to the set temperature.

Therefore, as described above with reference to FIG. 1 through FIG. 6, heat is supplied from the heat sources 5 to the reservoir tanks 4, and the liquids in the reservoir tanks 4 are respectively maintained at the high temperature (thermal denaturing temperature), the middle temperature (elongation reaction temperature) and the low temperature (annealing reaction temperature) by feedback control performed by the temperature sensors 16. The heat source is preferably located on a bottom surface of each reservoir tank 4 in order to effectively cause thermal convection since the temperature is to be controlled to a level higher than room temperature. The temperature of the liquid in each reservoir tank 4 is uniformized by a mechanism that uniformizes the temperature distribution of the liquid such as the stirring mechanism 6 or the like in addition to by the thermal convection. In this manner, the temperature is uniformized at higher speed. From each reservoir tank 4, the liquid is constantly discharged at a flow rate of, for example, 10 mL/sec. by the pump 6. When the liquid is not to be led to the heat exchange chamber 3, the liquid is refluxed by the switching valve 8 located on a stage after the pump to the auxiliary temperature control mechanism 10 via the bypass flow channel 9. After the temperature of the liquid is fine-tuned to the temperature set for the reservoir tank 4, the liquid is refluxed to the reservoir tank 4. Namely, the liquid is circulated from the reservoir tank 4 to the pump 7 to the switching valve 8 to the auxiliary temperature control mechanism 10 and back to the reservoir tank 4, so that the liquid is prepared. By contrast, when the temperature of the reaction vessel 1 is to be changed to the temperature of the liquid, the switching valve 8 on the stage after the pump 7 is switched such that the liquid is directed toward the heat exchange chamber 3 and also the switching valve 8 that controls the flowing direction of the liquid discharged from the heat exchange chamber 3 is switched. Thus, the liquid is circulated from the reservoir tank 4 to the pump 7 to the switching valve 8 to the heat exchange chamber 3 to the switching valve 8 to the auxiliary temperature control mechanism 10 and back to the reservoir tank 4. As a result, the temperature of the reaction vessel 1 is changed to the temperature of the liquid in a predetermined reservoir tank 4.

In this switching process, a slight difference in the amount of refluxing liquid is caused among the three reservoir tanks 4. Therefore, in the case where the three reservoir tanks 4 are controlled independently, a difference in the liquid surface level may be caused among the three reservoir tanks as the reaction process is repeated and as a result, a part of the tanks may be overflown with the liquid or a difference in the liquid transmission rate may be caused among the three reservoir tanks. In order to avoid these, a coupling tube 15 may be provided as an auxiliary mechanism that equalizes the liquid surface levels. The coupling tube 15 is provided for the purpose of equalizing the liquid surface levels but not for the purpose of actively transferring the liquids of different temperatures. Therefore, it is desirable that the coupling tube 15 is sufficiently thin. The coupling tube 15 is desirably located in the vicinity of the bottom surface of each reservoir tank 4.

An operation of the device according to the present invention in the case where a PCR is performed by use of the three-temperature reservoir tanks 4 will be described by way of a typical example, like the PCR performed with the two temperatures as shown in FIG. 6B. In this example, the three-temperature PCR is performed by the following steps. First, in order to cause an initial reaction, namely, thermal denaturation of double strand DNA on which the PCR is to be performed, a high-temperature liquid is refluxed for 10 seconds or longer from the high-temperature reservoir tank 4, in which the liquid temperature is set to 95°C or 96°C so that the temperature of the reaction vessel 1 is 94°C or higher. As a result, the temperature of the reaction vessel 1 is maintained at 94°C or higher for 10 seconds or longer. For setting the temperature for the thermal denaturation, the temperature of the high-temperature reservoir tank 4 is set such that even when there is a temperature distribution in the entire reaction vessel 1, the lowest temperature is 94°C or higher. As a result of the reflux, single strand DNA is produced, and an enzyme required for the PCR may be activated although whether the enzyme is activated or not depends on the type of enzyme. Next, an annealing step of specifically binding the primer and the DNA is performed as follows. A liquid is refluxed for 1 second from the low-temperature reservoir tank 4, in which the liquid temperature is set to 60°C so that the temperature of the reaction vessel is about 60°C. As a result, the temperature of the reaction vessel is made 60°C. In a final step, an elongation reaction of complementary DNA chain with heat-resistant DNA polymerase is performed as follows. A liquid is circulated for 3 seconds from the middle-temperature reservoir tank 4, in which the liquid temperature is set to 72°C so that the temperature of the reaction vessel 1 is about 72°C. When the elongation is finished, the step of the thermal denaturation is again performed. In this manner, the cycle of circulating the liquid of 95°C from the high-temperature reservoir tank 4 for 1 second to increase the temperature of the reaction vessel 1 to 94°C or higher, circulating the low-temperature liquid of 60°C for 1 second, and then circulating the middle-temperature liquid of 72°C for 3 seconds is repeated about 40 times. As a result, the DNA sequence area as the target can be amplified. In order to feed the liquids to the heat exchange chamber 3 so as not to actually cause a temperature drop of the liquids from the reservoir tanks 4, the liquids need to be refluxed at sufficiently high speed. In this example, it was possible to change the temperature at the circulation rate of the liquids of about 10 mL/sec., while preventing the temperature drop. Regarding the annealing step in this example, the optimal temperature at which the primer and the DNA are bound varies in accordance with the primer designed. Therefore, it is desirable that an optimal annealing temperature is calculated by use of melting curve analysis described later and the temperature of the low-temperature reservoir tank 4 is set to the resultant temperature. The time period for the elongation reaction is set to 3 seconds in this example, but is not limited to this. An appropriate time period may be calculated from the relationship between the elongation rate of the DNA polymerase enzyme and the target sequence size. For example, the DNA elongation rate by Taq polymerase, which is a representative DNA polymerase enzyme, is about 60 nucleotides/sec. at 70°C. Therefore, in the case where this enzyme is used, a target area of about 150 to 180 nucleotides can be amplified by an elongation reaction performed for 3 seconds. In order to effectively realize a high-speed PCR with this device, the target area is narrowed down to several hundred nucleotides to design a primer, and an optimal DNA polymerase enzyme is selected in accordance with the purpose. It is desirable to use a polymerase which reacts at higher speed.

In the case where the three-temperature cycle is repeated, at least two measurement methods, specifically, an end-point measurement method and a real-time amplification measurement method, can be combined. As described above with reference to FIG. 1, the high-speed gene amplification device according to the present invention allows incorporation of an optical detection system that optically monitors amplification of a target gene product. For a method which uses a fluorescent dye, generally referred as an "intercalator type fluorescent dye", that has the fluorescence intensity thereof significantly changed when being incorporated into a hydrogen bonding area of double strand DNA, it is important that the target DNA product should be of double strand in order to quantitatively measure the amount of the product. Therefore, in the case where the end-point measurement method, by which the measurement is performed after the reaction is finished, is used, it is desirable that the measurement is performed when the product is stabilized in a temperature range where the product can be measured in a double strand DNA state after the reaction is finished, or that the fluorescence intensity is measured immediately after the final amplification cycle is finished and the resultant fluorescence intensity is compared for analysis with the pre-amplification reaction fluorescence intensity to estimate the magnitude of the fluorescence intensity amplified by the double strand DNA. It is important that the measurements should be performed at the same temperature because of the thermal fluorescence quenching phenomenon, by which the fluorescence intensity of a fluorescent dye in a solution varies in accordance with the temperature of the solution.

By contrast, with the real-time measurement method, the amplified magnitude is estimated for each amplification cycle. Therefore, the measurement needs to be performed in each cycle. Desirably, the measurement in each cycle is performed when the elongation reaction is almost over and the thermal denaturation is about to start. In this case also, it is desirable that the temperature of the solution is the same among the cycles in order to eliminate the influence of the thermal fluorescence quenching phenomenon. The measurement may be performed by use of a method generally referred to as the TaqMan^{®} probe method. According to this method, DNA polymerase having a 5'-3' exonuclease function, and probe DNA fragment containing a donor fluorescent dye and an acceptor fluorescent dye are used in order to respond to the fluorescent energy transfer. With this measurement method, the 5'-3' exonuclease reaction advances during the elongation reaction of the DNA polymerase. Therefore, in the case where the end-point measurement method is used, fluorescence intensities of the donor fluorescent dye and the acceptor fluorescent dye are measured before the gene amplification reaction is performed at the respective fluorescent wavelengths. After the gene amplification reaction is finished, fluorescence intensities of the donor fluorescent dye and the acceptor fluorescent dye are measured at the respective fluorescent wavelengths to quantitatively detect how much of the probe DNA has actually been decomposed by the enzyme. In this manner, it can be analyzed whether the target nucleotide sequence is present or absent. In this case also, it is desirable that the measurements are performed at the same solution temperature in order to eliminate the influence of the thermal fluorescence quenching phenomenon. By contrast, with the real-time measurement method, the decomposition reaction of the probe DNA fragment advances during the elongation reaction of the polymerase. Therefore, the fluorescence intensities of the donor fluorescent dye and the acceptor fluorescent dye may be measured at the respective wavelengths when the elongation reaction is finished, at the time of thermal denaturation, or at the time of annealing in each amplification cycle. It should be noted that in this case also, it is desirable that the temperature of the solution is the same among the cycles in order to eliminate the influence of the thermal fluorescence quenching phenomenon.

The reaction vessel 1 used for the high-speed PCR according to the present invention may be a disposable chip. In this case, the reaction vessel 1 is replaced with a new one as follows. The liquid filling the heat exchange chamber 3 is discharged until no liquid remains in the heat exchange chamber 3. In this state, the reaction vessel casing 2 is detached, and then the reaction vessel 1 is detached. In the example shown in FIG. 13, an air inlet tube 2001 provided with a valve (switching valve) 8 is located so as to be connected to the heat exchange chamber 3; and a discharge tube 2002 for the liquid in the heat exchange chamber 3, that is provided with a valve (switching valve) 8 and a liquid discharge pump 6, is located so as to be connected to a circulation channel leading to the auxiliary temperature control mechanism 10 for one of the reservoir tanks 4. During the gene amplification reaction or the like actually performed by use of the liquids in the three-temperature reservoir tanks 4, the two valves 8 provided for the tubes 2001 and 2002 are closed, and thus the tubes 2001 and 2002 do not influence the reflux of the liquids from the three reservoir tanks 4. At the time when the gene amplification reaction is finished, the six switching valves 8 connected to the three reservoir tanks 4 are switched to prevent the liquids from flowing between the reservoir tanks 4 and the heat exchange chamber 3. Then, the valves 8 respectively provided for the tubes 2001 and 2002 are connected to the tubes 2001 and 2002, and the liquid in the heat exchange 3 is fed to, for example, the auxiliary temperature control mechanism 10 for the low-temperature reservoir tank 4 by use of the pump 7. Thus, the heat exchange chamber 3 is filled with air. After this, the reaction vessel 1 is replaced. When a new reaction vessel 1 is placed, the switching valves 8 connected to the tubes 2001 and 2002 are closed to start feeding the liquid through the reflux system from one of the reservoir tanks 4. As a result, the heat exchange chamber 3 is filled with the liquid, and the usual gene amplification reaction can be restarted.

With the device according to the present invention, a mechanism that controls the liquid temperature can be used to perform a melting curve analysis. The melting curve analysis may be performed as follows, for example. The reaction liquid in the reaction vessel 1 is changed at a ramp rate of 0.11°C/sec. continuously from 65°C to 95°C. While the temperature of the reaction vessel 1 is monitored by a liquid temperature sensor placed in the reaction vessel 1, change in the fluorescent intensity of the intercalator fluorescent dye in the PCR liquid contained in the reaction vessel 1, for example, is measured by an optical measurement module as shown in FIG. 1. Thus, a melting curve analysis on the correlation between the temperature and the fluorescence intensity can be performed. In this process, for example, one of the reservoir tanks provided with the auxiliary liquid heat release mechanism 17, among the three reservoir tanks 4 shown in FIG. 13, is used to first decrease the temperature of the liquid in the reservoir tank to a predetermined start temperature on a low-temperature side. Next, heat is provided to the heat source in the reservoir tank little by little. While the temperature is measured by the temperature sensor 16, the heat is provided in such an amount as to increase the temperature at a rate of about 0.11°C/sec. At the same time, the liquid in the reservoir tank is supplied to the heat exchange chamber 3. The liquid returned from the heat exchange chamber 3 is adjusted by the auxiliary temperature control mechanism 10 to have the same temperature as that of the reservoir tank and then is refluxed to the reservoir tank. In a final step, when the temperature in the reaction vessel 1 reaches a final temperature of, for example, 95°C, the temperature of the liquid in the reservoir tank is decreased by the auxiliary liquid heat release mechanism 17 down to the initially set level.

As described above with reference to FIG. 1, the reaction vessel 1 is maintained airtight by the reaction vessel casing 2. In a preferable embodiment, the reaction vessel casing 2 is constantly maintained at a temperature of 75°C or higher by a heater incorporated into the reaction vessel casing 2 based on the temperature data acquired by the temperature sensor 16. The reaction vessel casing 2 is heated by the heater to prevent the water vapor in the casing from condensing on an inner surface thereof. This maintains the pressure of the water vapor in the casing at a certain level. As a result, the liquid droplets in the reaction vessel can have the temperature thereof changed in the range of 50 to 97°C without being evaporated even in an exposed state with no additive such as mineral oil or the like.

Similarly, the temperature control technique for the melting curve analysis may also be used to maintain the temperature at a certain level different from the temperature for the PCR. This allows a reverse transcription reaction to be performed on the PCR liquid contained in the reaction vessel as follows, for example. A liquid of 50°C is refluxed for 10 minutes or longer to transcribe RNA to DNA, and then the temperature of the reservoir tank is adjusted to a level at which a usual PCR is performed. In this manner, the reverse transcription reaction and the PCR can be performed successively.

As specific methods for performing a gene amplification reaction successively after the reverse transcription reaction, a one-step operation (by which reverse transcription and amplification are performed successively in one tube) and a two-step operation (by which reverse transcription and amplification are performed in different tubes) are available. Herein, the one-step operation will be described as an example. As a reverse transcription enzyme for a one-step RT-PCR performed on a short target, Tth DNA polymerase (Roche), for example, may be used. With this polymerase, the one-step operation is performed as described in (1) through (3). The optimal temperature for the reaction is 55 to 70°C. (1) The temperature of the low-temperature reservoir tank is set to 50 to 60°C, which is lower than the usual annealing temperature, and a liquid in the low-temperature reservoir tank is refluxed to the heat exchange chamber 3 for about 30 minutes to perform a reverse transcription. (2) Next, a liquid having a temperature of 94°C or higher is refluxed from the high-temperature reservoir tank 4 to the heat exchange chamber 3 for about 2 minutes to perform initial thermal denaturation. (3) Then, the following amplification cycle is performed. A thermal denaturation process is performed for 1 second or longer with a liquid having a temperature of 94°C or higher from the high-temperature reservoir tank 4. Then, an annealing process is performed with a liquid having a temperature of 45 to 66°C corresponding to the primer characteristics. The liquid is from the low-temperature reservoir tank 4, which has been temperature-adjusted for the annealing. Then, an elongation reaction is performed for 3 seconds with a liquid having a temperature of 68 to 70°C corresponding to the enzyme characteristics. The liquid is from the middle-temperature reservoir tank 4. As a result of performing such a cycle, the target RNA can be amplified. In this example, the temperature of one of the three reservoir tanks 4 having different temperatures is adjusted to a temperature optimal for the reverse transcription reaction, and the reverse transcription reaction is performed; and then the temperature of the same reservoir tank 4 is set again to a temperature optimal for the PCR, and the three-temperature gene amplification reaction is performed. Alternatively, a fourth reservoir tank 4 having a temperature optimal for the reverse transcription may be provided to perform the above-described process.

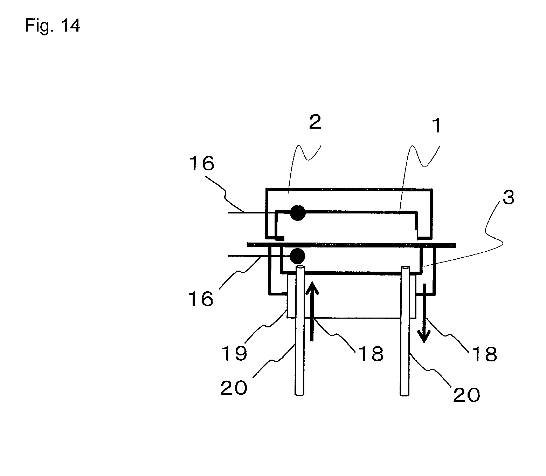

FIG. 14 shows one example of another technique for providing the reaction vessel 1 with a measurement function for the above-described melting curve analysis. In this example, a Peltier temperature control mechanism 19 is located on a bottom surface of the heat exchange chamber 3. Since flow channel tubes 20 for a liquid to be circulated for a PCR run through the Peltier temperature control mechanism 19, the liquid for the high-speed PCR can be circulated as described above with reference to FIG. 13. For performing a measurement for the melting curve analysis, flows 18 of the liquid in the flow channel tubes 20 are stopped and the temperature of the still liquid filling the heat exchange chamber 3 is controlled by the Peltier temperature control mechanism 19 and the temperature sensor 16 located in the heat exchange chamber 3. Thus, the melting curve analysis is performed. This mechanism is also usable for a reverse transcription reaction as follows. A temperature and a time period optimal for the reverse transcription reaction are provided by the Peltier temperature control mechanism 19. When the reverse transcription reaction is finished, a high-speed gene amplification can be performed successively by use of the structure of the two-temperature gene amplification device as shown in FIG. 1 or the structure of the three-temperature gene amplification device as shown in FIG. 13.

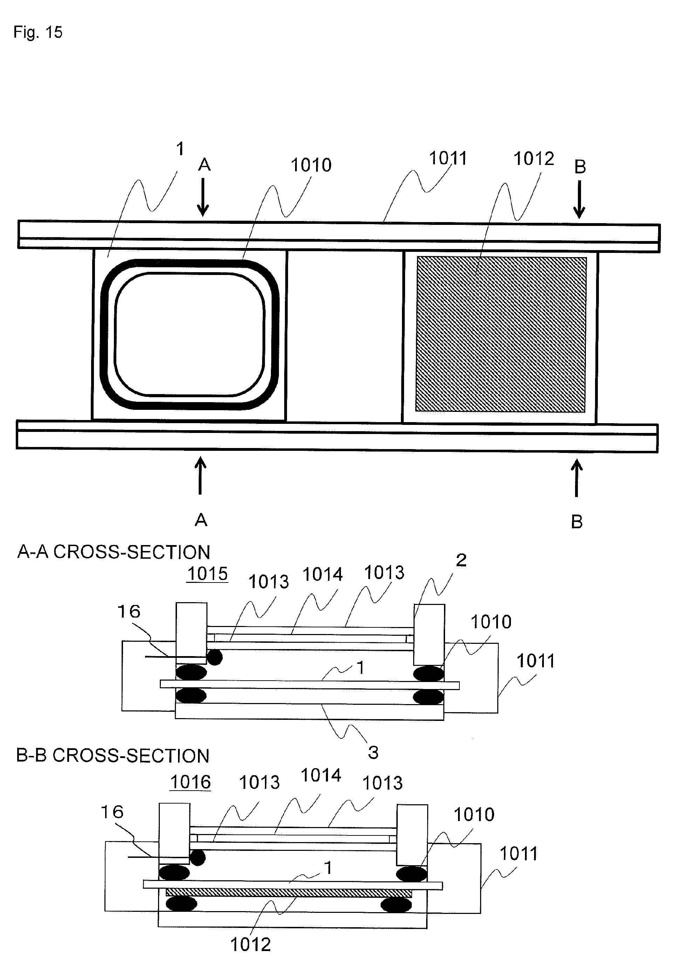

FIG. 15 provides schematic views showing an exemplary embodiment of the present invention in which disposable reaction vessels 1 are used successively, and also an exemplary embodiment of a reaction part including the reaction vessel 1 on which a reverse transcription reaction is performed. As described above with reference to FIG. 13, the reaction vessel 1 according to the present invention is usable as a disposable chip. In this case, as shown in a cross-sectional view taken along line A-A in FIG. 15, a reaction vessel part 1015 that performs a high-speed gene amplification reaction typically includes, for example, the reaction vessel 1 for performing a PCR, a reaction vessel casing 2 and a heat exchange chamber 3 that hold the reaction vessel 1 at top and bottom surfaces of the reaction vessel 1, and a guide rail 1011 for transporting the reaction vessel. Spacers that have guaranteed heat insulation property and sealability and secure the reaction vessel 1, such as O-rings 1010 or the like, are provided between the reaction vessel casing 2/the heat exchange chamber 3 and the reaction vessel 1. With this structure, a plurality of the reaction vessels 1 can be transported to the reaction vessel part 1015 along the guide rail 1011. When the reaction vessel 1 is to be used for a reaction, the reaction vessel 1 is held between the reaction vessel casing 2 and the heat exchange chamber 3 and thus secured by the O-rings 1010. In this state, a liquid can be introduced to the reaction vessel 1 from the liquid reservoir tank 4. When the reaction vessel 1 is to be transported, the reaction vessel casing 2 and the heat exchange chamber 3 are detached from the reaction vessel 1 and the O-rings 1010 are loosened. In this state, the reaction vessel 1 as a chip can be moved along the guide rail 1011 and replaced with another reaction vessel. As can be seen from the cross-sectional view taken along line A-A in FIG. 15, the reaction vessel casing 2 may be optically transparent in order to optically observe and measure the reaction liquid located on the reaction vessel 1. In this case, a transparent electrode 1014 which is formed of ITO or the like and effectively generates heat by resistance is held between two thin glass plates 1013, so that the temperature of the reaction vessel casing 2 can be controlled without hindering optical observation. Especially because a temperature sensor 16 is located on an inner surface of the inner glass plate 1013 and is adjusted to have a temperature of 75°C or higher, the liquid is prevented from condensing on an inner surface of the reaction vessel casing 2 and thus the water vapor pressure can be prevented from changing. As a result, the reaction liquid located on the reaction vessel 1 can be prevented from evaporating.

A reverse transcription reaction vessel part 1016 is provided on the guide rail 1011, on a stage before the reaction vessel part 1015. This allows a reverse transcription of an RNA sample to cDNA so that a high-speed gene amplification can be performed in the reaction vessel part 1015 on a later stage. As can be seen from a cross-sectional view taken along line B-B in FIG. 15, the reverse transcription reaction vessel part 1016 includes a casing having a structure similar to that of the reaction vessel casing 2, and this casing may be optically transparent in order to optically observe and measure the reaction liquid located on the reaction vessel 1. In this case, a transparent electrode 1014 which is formed of ITO or the like and effectively generates heat by resistance is held between two thin glass plates 1013, so that the temperature of the casing can be controlled without hindering optical observation. Especially because a temperature sensor 16 is located on an inner surface of the inner glass plate 1013 and is adjusted to have a temperature of 75°C or higher, the liquid is prevented from condensing on an inner surface of the casing and thus the water vapor pressure can be prevented from changing. As a result, the reaction liquid located on the reaction vessel 1 can be prevented from evaporating. On a bottom surface of the reaction vessel 1, a reverse transcription reaction temperature plate 1012 is located. In the state of being set to, for example, 50°C as a temperature optimal for a reverse transcription, the reverse transcription reaction temperature plate 1012 is brought into close contact with the reaction vessel 1. Thus, the PCR solution on the reaction vessel 1 is reacted for about 30 minutes while the temperature of the plate contacting the reaction vessel 1 is 50°C to perform a reverse transcription reaction. When the reverse transcription reaction is completed, the reaction vessel 1 is slid along the guide rail and transferred to the reaction vessel part 1015, where a PCR is started.

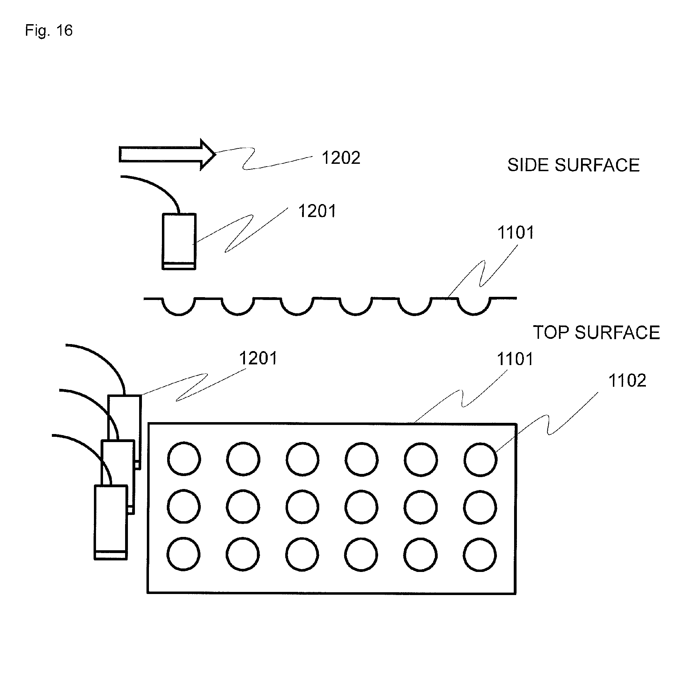

FIG. 16 provides schematic views showing an exemplary embodiment of a real-time detection method according to the present invention for a multiple sample gene amplification reaction. A reaction detection device according to the present invention typically includes a multi-well reaction vessel 1101, a plurality of reaction wells 1102 arranged in an array, and a detector 1201. During a PCR, the detector 1201 performs a scan in, for example, a direction 1202 to detect the fluorescence intensities of the PCR samples in the reaction wells 1102. In this manner, the fluorescence intensities of all the samples are measured with detectors provided in a number smaller than the number of the reaction wells. As described above with reference to FIG. 13, for performing fluorescence detection by use of an intercalator system, it is desirable to perform the measurement while a double strand DNA state is maintained after the PCR elongation reaction is finished but before the thermal denaturation is started. In the case where a method such as the TaqMan^{®} probe method or the like, by which a fluorescent probe amount that is changed at the time of the PCR elongation reaction is detected, is used, the measurement can be performed at any time after the PCR elongation reaction is finished, namely, either on the stage of the thermal denaturation or on the stage of the annealing. It should be noted that regardless of when the measurement is performed, it is desirable that the reaction liquids containing the fluorescent dye have the same temperature in order to compare the measured fluorescence intensities, for the following reason. Due to the temperature dependence of fluorescence quenching, even the fluorescent dye contained in the same reaction liquid may exhibit a different fluorescence intensity when the temperature is different.

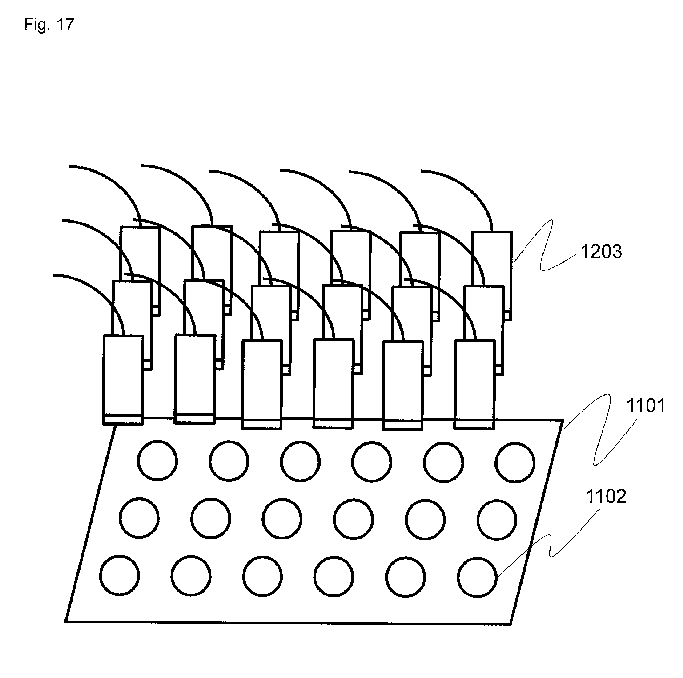

FIG. 17 is a schematic view showing an exemplary embodiment of a real-time detection method according to the present invention for a multiple sample gene amplification reaction. A reaction detection device according to the present invention typically includes a multi-well reaction vessel 1101, a plurality of reaction wells 1102 arranged in an array, and detection probes 1203. The detection probes 1203 in the same number as that of the reaction wells 1102 provided on the multi-well reaction vessel 1101 are prepared and arranged in an array, and a fixed point observation is made on the fluorescence intensities of PCR samples to measure continuous time-wise change. Unlike in the scan-type device shown in FIG. 16, continuous change in the fluorescence intensity can be detected.

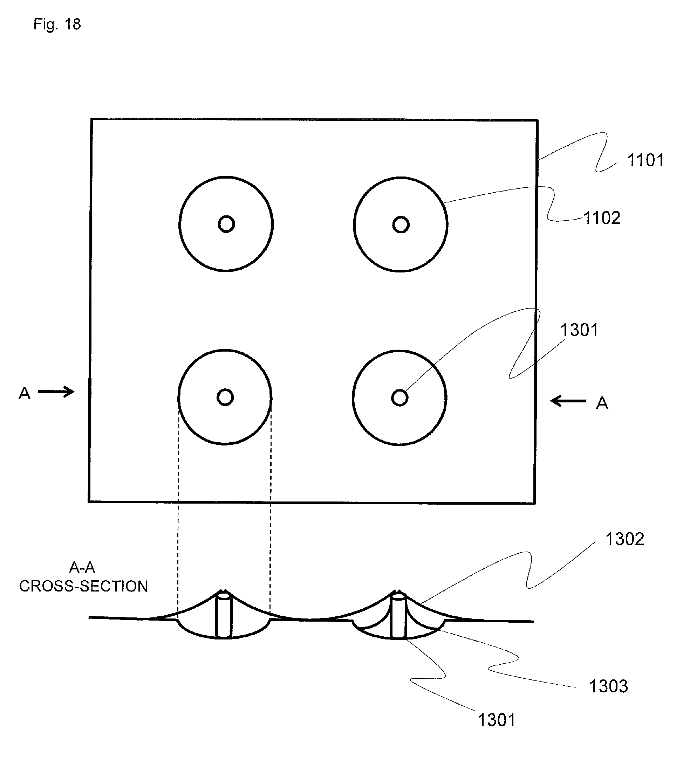

FIG. 18 schematically shows an exemplary embodiment in which seals 1302 are pasted on the reaction vessel 1101 so as to enclose and prevent a sample reaction liquid in reaction wells 1102 from evaporating and thus to prevent the reaction vessel 1 from being dried during a PCR. In this example, a pillar 1301 is provided at the center of each reaction well in order to prevent the seal 1302 from contacting a sample solution 1303 in the reaction well 1102 and also in order to suppress the sample solution from moving in the reaction well during dripping of the reaction solution, during the transportation or during the PCR measurement. The pillar 1301 may be formed by punching a metal plate of aluminum or the like having high heat conductivity or may be formed of glass or a plastic material which is optically transparent.

The above-described structure prevents the PCR solution in an amount of 5 to 10 µL from moving and also from contacting the sealant during the PCR. In addition, the pillar allows the liquid droplets to be spread in a wider area. As compared with the case where droplets of the reaction liquid are merely dripped to a surface of the reaction vessel 1101, the reaction liquid can be spread in a wider area. Thus, the temperature of the reaction liquid can be transferred to the heat exchange chamber more efficiently. In the case where the pillar 1301 is formed of an optically transparent plastic material or a material having a polymeric structure such as PDMS or the like, a substance which generates fluorescence having a wavelength different from the wavelength detected during the real-time PCR measurement may be kneaded into the pillar, so that the pillar 1301 can be used as reference for calibration of the fluorescence intensity of the PCR.

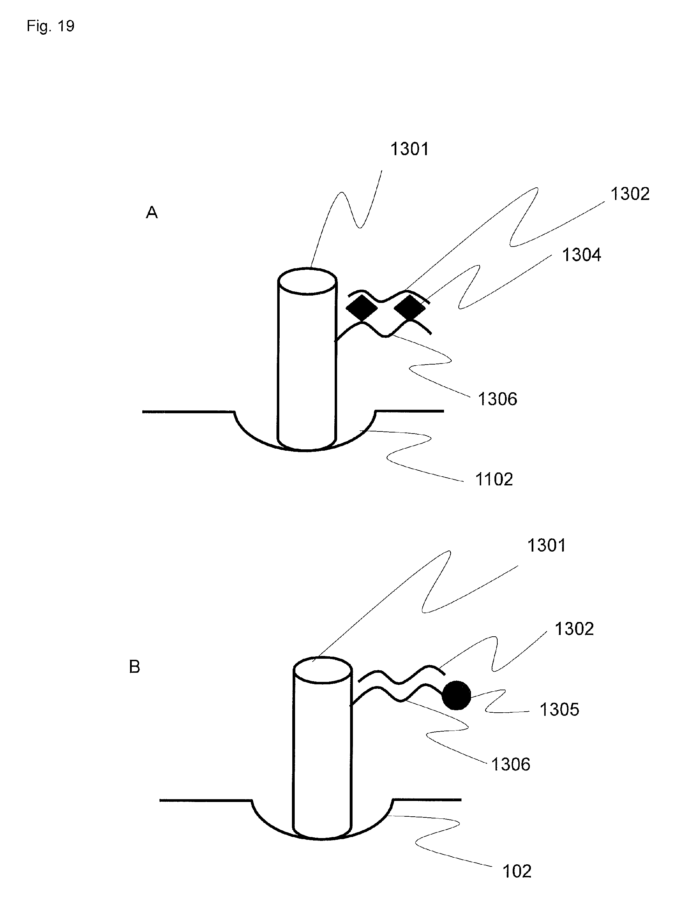

FIG. 19 provides schematic views showing an exemplary embodiment in which a DNA probe 1306 or a primer is bound to a surface of the pillar 1301 formed of optical fiber glass, a plastic material or the like that has optical conductivity and is placed in the reaction well 1102, so that the DNA which is being subjected to the PCR can hybridize in the vicinity of the pillar 1301. With this structure, in the case where amplification of a PCR reactant is effectively performed, the DNA amplification products are effectively bound in the vicinity of the surface of the pillar, and the fluorescence of the DNA amplification products can be detected utilizing the optical fiber characteristics of the pillar to perform real-time detection of the PCR at a detection sensitivity higher than usual. Usable as a mechanism that generates fluorescence is, for example, a mechanism that uses an intercalator 1304 such as SYBR Green or the like, which intercalates to double strand DNA to generate fluorescence as shown in FIG. 19A, or a probe method using a fluorescence resonance energy transfer (FRET) method by which, as shown in FIG. 19B, when a PCR reactant 1302 is hybridized, the three-dimensional structure of the DNA is destroyed and thus a fluorescence substance 1305 bound to a terminus of the DNA probe generates fluorescence. Alternatively, in the case where the target DNA is effectively amplified, an acceptor fluorescent dye is introduced into a surface or the inside of the pillar 1301 and an intercalator is used as a donor fluorescent dye, so that the probe DNA bound to the surface of the pillar and the amplification product DNA are bound together, and the intercalator fluorescence is effectively generated on the surface of the pillar. Using this, the acceptor fluorescence can be measured based on the fluorescence emission from the pillar. This has the following advantage. Unlike by the conventional observation of the target DNA using the change in the fluorescence intensity of the intercalator as an index, the fluorescence having a wavelength different from that of the intercalator fluorescent dye, specifically, acceptor fluorescence emission information, can be observed owing to the transfer of the fluorescence energy. This allows quantitative measurement. The quantitative measurement is made possible at lower noise.

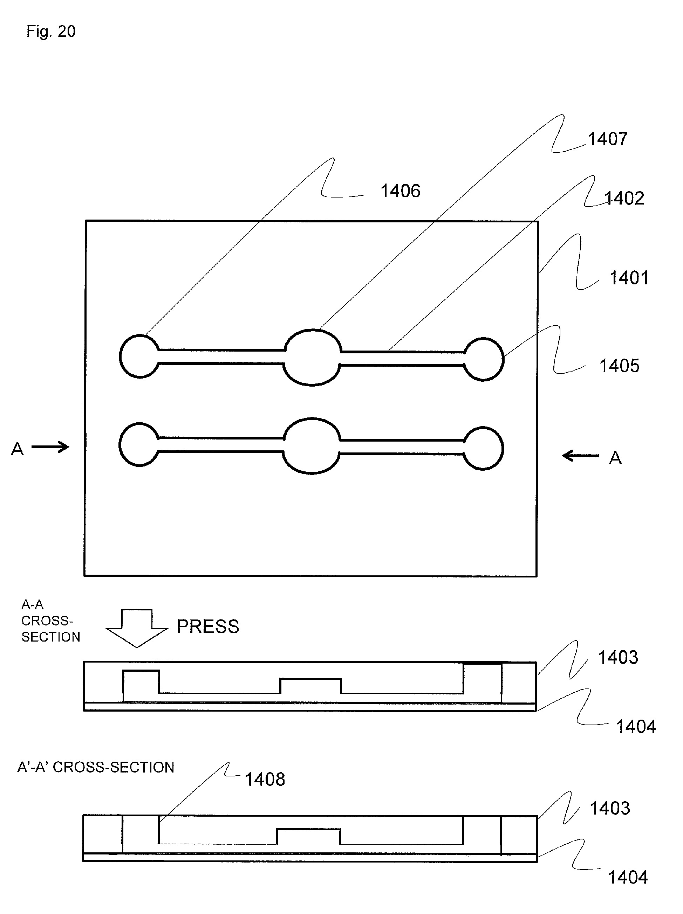

FIG. 20 schematically shows an example in which an introduction channel for a reaction liquid and a reaction area are formed in the reaction vessel by a microprocessing technology. Such a structure is provided in order to locate the reaction liquid in a space in the reaction vessel 1101 so as to effectively control the volume of the reaction liquid and allow the reaction liquid to contact the heat exchange chamber with a larger surface area, which is not realized by merely the conventional technique of dripping the liquid droplets. A micro flow channel type reaction vessel 1401 shown in this example includes a reaction vessel 1404 formed of an aluminum thin plate or the like which is used in the examples shown in FIG. 1 through FIG. 19 and has high heat conductivity, and a flow channel-forming polymer 1403 formed of a material which is optically transparent and elastic such as polydimethylsiloxane (PDMS) or the like. The flow channel-forming polymer 1403 is pasted on the reaction vessel 1404. This chip includes a sample injection opening 1405, a micro flow channel 1402 in which a reaction liquid introduced via the sample injection opening flows by capillary action, a reaction liquid reservoir 1407 to be filled with the reaction liquid, and an air reservoir 1406 that is to be pressed to recover the reaction liquid via the sample injection opening 1405 by air pressure. In this example, after the reaction liquid is introduced via the injection opening 1405, gene amplification is performed by use of the high-speed gene amplification device shown in FIG. 1 or FIG. 13. Then, the air reservoir 1406 for sample recovery can be pushed to recover the reaction liquid via the sample injection opening 1405. An appropriate volume of the reaction solution to be fed is, for example, 5 µL or smaller, and it is desirable to suppress the capacity of an area from the injection opening 1405 to the reaction liquid reservoir 1407 to 5 µL or smaller. In this example, the air reservoir is used. Alternatively, as shown in a cross-sectional view taken along line A'-A' in FIG. 20, a sample discharge opening 1408 may be provided at the position of the air reservoir. In this case, air can be blown via the sample injection opening 1405 to recover the reaction liquid via the sample discharge opening 1408. In the example shown in FIG. 20, the reaction liquid reservoir 1407 has a greater height than that of the micro flow channel 1402. In order to perform heat exchange more effectively, it is preferable that the reaction liquid reservoir 1407 is made as low as possible to increase the planar area thereof. In this manner, the PCR can be performed more efficiently.

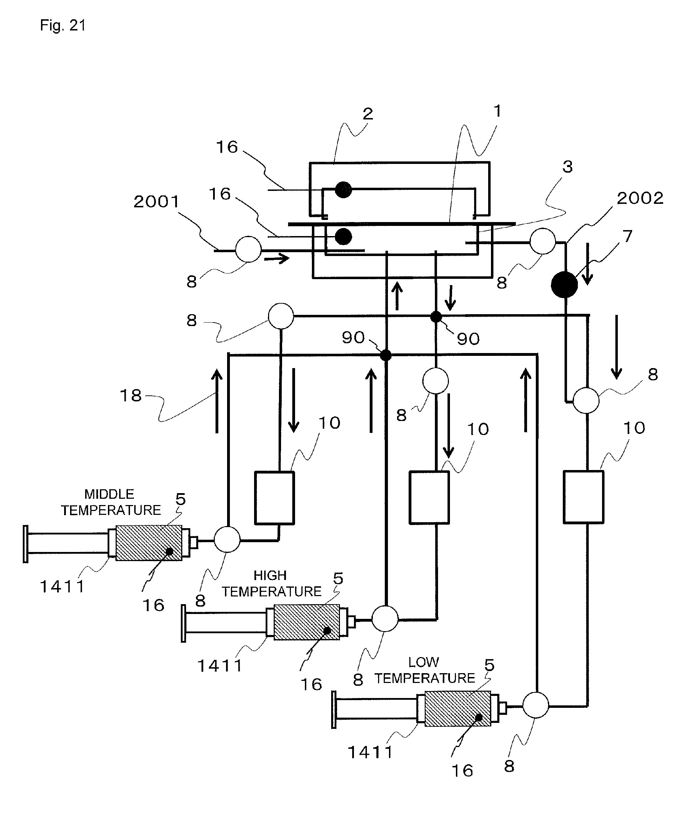

FIG. 21 schematically shows an exemplary embodiment which is different from the example shown in FIG. 13 in that syringe pumps 1411 are used as a mechanism that feeds a liquid for temperature control. The syringe pumps 1411 are capable of automatically feeding and absorbing a liquid at a flow rate of 10 mL/sec. or higher. The heat source 5 is located on a surface of each syringe pump 1411, and the temperature sensor 16 is located in each syringe pump 1411. For introducing a liquid for temperature control into the heat exchange chamber 3, the switching valve 8 for the syringe pump 1411 corresponding to the liquid of the temperature to be introduced is switched, such that the liquid is introduced from the corresponding syringe pump to the heat exchange chamber 3 via the joint 90. The liquid returning from the heat exchange chamber 3 is stored in the auxiliary temperature control mechanism 10 via the joint 90 and the switching valve 8, and is refluxed to the syringe pump after the temperature of the liquid is returned to the temperature set for the syringe pump 1411. When the feeding of the liquid from the above syringe pump is finished and a liquid of another temperature is started to be fed from another syringe pump 1411, the switching valve 8 is switched to connect the above syringe pump 1411 and the auxiliary temperature control mechanism 10 so that the liquid is recovered to the syringe pump 1411.

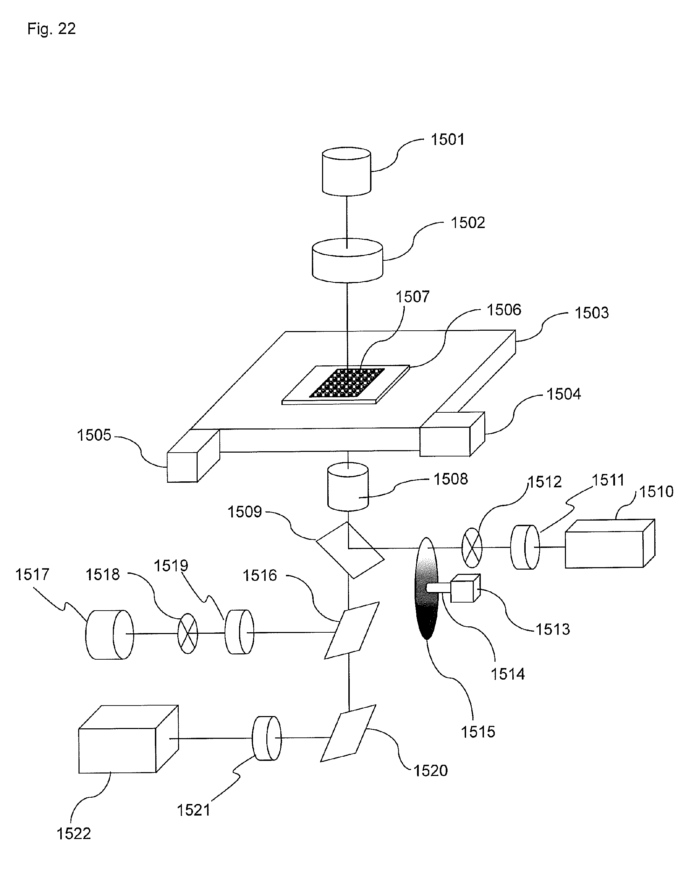

FIG. 22 shows an example in which the reaction liquid is irradiated with infrared rays, which are highly absorbed by water as the reaction liquid, so as to control the temperature of the reaction liquid by use of the temperature change caused by the absorption. The present inventors have already described a high-speed PCR device technology using the absorption of converged infrared light in Japanese Laid-Open Patent Publication No. 2008-278791. In the example shown in FIG. 22, the temperature of the reaction liquid is not controlled by use of the intensity of light from an infrared laser 1510 but is controlled by use of a gradation ND filter 1515 and a motor 1513 such as a stepping motor or the like that precisely controls the angle of the ND filter 1515 by use of a shaft 1514. The transmittance of light through the ND filter 1515 is kept changed stepwise while the ND filter 1515 is rotated. The angle of the ND filter 1515 is changed at high speed to change the intensity of the light at high speed. This realizes a rapid temperature change. In addition, the gradation ND filter 1515 may be rotated at various angular velocities, so that the temperature gradient of the reaction liquid per unit time can be controlled accurately and precisely, and also the temperature change can be programmed in any manner.

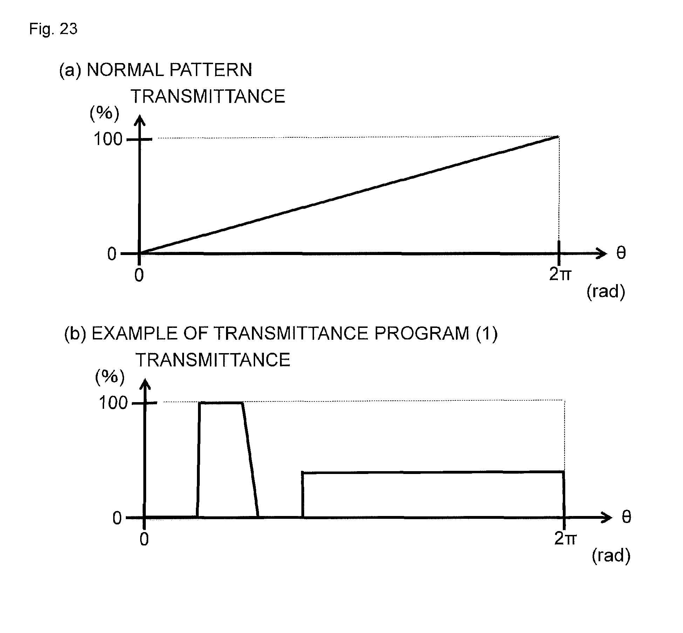

The device shown in FIG. 22 has the following structure. Visible light from an illumination light source (halogen lamp, etc.) 1501 is collected by a condenser lens 1502. Each of reaction liquids on a reaction well plate 1507 located on an automatic XY stage 1503 is focused on by an objective lens 1508 so that the state thereof can be observed by an image observation camera (cooled CCD camera, etc.) 1522. The automatic XY stage 1503 is driven by an X-axis motor 1504 and a Y-axis motor 1505 so that a well at a desired coordinate position can be observed. A stage heater 1506 controls the temperature of the reaction well plate 1507 to the lowest temperature in the PCR such as, for example, the annealing temperature or the like. The infrared laser 1510 is structured to introduce light to a microscope optical system by an infrared laser dichroic mirror 1509 via a beam expander 1511, a laser shutter 1512 and the gradation ND filter 1515. The shaft 1514 at the center of the gradation ND filter 1515 is connected to the motor 1513 such as a stepping motor or the like, and thus the transmittance of infrared laser light through the gradation ND filter 1515 can be freely adjusted. Referring to FIG. 23(a), a surface of the gradation ND filter 1515 may usually have an ND gradation pattern that changes linearly in accordance with the angle θ in the relationship of ND=ND_{MAX}·(θ/θ_{MAX}). Alternatively, the gradation pattern may be pre-written in accordance with the angle θ, so that the water droplets can be irradiated with infrared rays having an intended intensity while the gradation ND filter 1515 is rotated at a certain angular velocity. FIG. 23(b) shows an example in which the gradation pattern is arranged such that one cycle of the usual process of three-temperature PCR is performed while the gradation ND filter 1515 is rotated once. First, the transmittance is rapidly raised from 0% to 100% to raise the temperature of the water droplets to 95°C or higher, so that the thermal denaturation of nucleic acid is performed. Next, the external temperature is set to the annealing temperature of about 55 to 60°C in advance and the transmittance is returned to 0%, so that the temperature of the water droplets is decreased to the annealing temperature. Then, the discoidal ND filter is rotated to allow, for example, about 30% of the light to be transmitted. Thus, the temperature of the water droplets is changed to 70°C. In this manner, the PCR elongation reaction advances. When the discoidal ND filter is to be rotated at a certain angular velocity, a desired ratio of the irradiation time periods for various transmitted lights can be realized by spatially arranging the gradation pattern such that the arrangement reflects the ratio. In order to allow fluorescence observation for quantitative measurement of the PCR in the reaction wells, this optical system is structured to introduce exciting light from a fluorescence-exciting light source (mercury lamp, etc.) 1517 by a fluorescence-exciting light dichroic mirror 1516 via a fluorescence-exciting light source shutter 1518 and a fluorescence-exciting light transmitter lens 1519. Quantitative measurement of the fluorescence intensity can be performed by an image observation camera 1522 such as a cooled CCD camera or the like via a camera dichroic mirror 1520, adjusted so as to block light having a wavelength from a heating infrared laser and also block the exciting light, and an imaging lens 1521. The optical heating technique shown in FIG. 22 can be flexibly combined with any of the techniques shown in FIG. 1 through FIG. 21.

For example, any of the examples shown in FIG. 1 through FIG. 21 may be combined with the optical heating technique shown in FIG. 22, so that the function of melting curve analysis can be provided easily with no need to change the temperatures of the reservoir tanks 4. Specifically, a liquid having the lowest temperature among the liquids to be circulated at high speed from the reservoir tanks 4 is circulated in advance, or water droplets containing a target nucleic acid molecule and a fluorescent dye having an intercalator function are continuously irradiated with heating infrared rays before the introduction of the high-speed circulating liquid is started. In this process, the gradation ND filter 1515 having a gradation pattern that decreases the light transmittance linearly is rotated at such an angular velocity that the temperature of the water droplets adequately stably follows the increase or decrease of the transmittance realized by the gradation pattern. Thus, the temperature of the water droplets can be increased or decreased linearly. The temperature of the water droplets is increased when the gradation ND filter 1515 is rotated in a direction in which the value ofND is decreased, and is decreased when the gradation ND filter 1515 is rotated in a direction in which the value ofND is increased. Information on the angle of the gradation ND filter 1515 and the change in the fluorescence intensity are recorded while the gradation ND filter 1515 is rotated at a low rate of 0.1 radians/sec. or lower, and in this state, a method for estimating the temperature of liquid droplets based on the angle θ described below is used. As a result, the temperature of the liquid droplets and the fluorescence intensity can be acquired, and thus the melting curve analysis can be performed. The method for estimating the temperature is as follows. A transmittance of 0% is set as θ=0 radians and a transmittance of 100% is set as θ=2π radians. The intensity of the infrared rays from the light source is adjusted such that the water droplets have a temperature of 95°C or higher when being irradiated with light having a transmittance of 100%. While the adjusted fluorescence intensity is maintained, the gradation ND filter is rotated. The temperature of the water droplets can be estimated by measuring the angle θ by use of the expression of (θ/2π)·(T_{MAX}-T_{MIN}). In the expression, T_{MAX} is the temperature of the water droplets when the water is irradiated with light having a transmittance of 100%, and T_{MIN} is the temperature of the water droplets when the water is irradiated with light having a transmittance of 0%.

Alternatively, as shown in FIG. 23(b), the ratio among transmittances of the light through the ND filter may be set in advance on the discoidal gradation ND filter in accordance with θ such that during one rotation of the gradation ND filter, one or several cycles of PCR are performed, on the premise that the gradation ND filter is rotated at a certain angle θ velocity. In this manner, a high-speed single reflux system that refluxes a high-speed liquid having a temperature that stabilizes the intended lowest temperature of the liquid droplets can be combined with the structure in this example. In this case, a high-speed PCR can be easily realized by photothermal conversion. Since there is only one high-speed reflux system, it is not necessary to incorporate the complicated switching valves or switching programs described above with reference to FIG. 1 through FIG. 21. It is also sufficient to provide only one reservoir tank. This significantly simplifies the structure.

The present invention also provides a device for gene analysis or gene amplification. This device includes a reaction vessel including one or a plurality of wells for accommodating a sample liquid containing nucleic acid; a heat sink or a heat exchange chamber; a temperature controller (e.g., temperature sensor, heating wire, liquid reflux type temperature controller), for the heat sink or the heat exchange chamber, that is structured to keep a temperature of the heat sink or the heat exchange chamber at a first temperature (e.g., nucleic acid elongation reaction temperature); a thermoelectric element (e.g., Peltier element) that is located between the heat sink or the heat exchange chamber and the reaction vessel and acts as a medium for heat transfer between the heat sink or the heat exchange chamber and the reaction vessel; and a thermoelectric element controller (e.g., temperature sensor, computer (e.g., PC)) that changes the temperature of the reaction vessel between the first temperature and a second temperature (e.g., nucleic acid denaturation temperature or annealing temperature) by controlling the operation of the thermoelectric element. The device is structured such that the temperature of the reaction vessel is the first temperature when the operation of the thermoelectric element is in an off state.

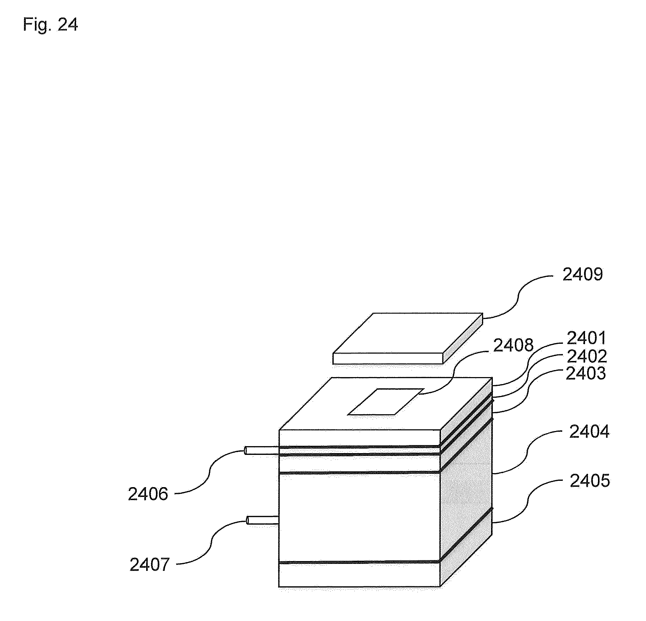

FIG. 24 is a schematic view showing a non-limiting example of structure of such a device for gene analysis or gene amplification according to the present invention. An embodiment according to the present invention shown in FIG. 24 includes a DNA reaction chip container 2401 that holds a sample containing DNA, a Peltier element 2403, a heat conductive thin plate 2402 for raising the heat conduction efficiency between the Peltier element 2403 and the reaction chip container 2401, a heat sink 2404, a heating wire 2405 for heating the heat sink 2404, a temperature sensor 2406 for monitoring the temperature of the reaction chip container 2401, a temperature sensor 2407 for monitoring the temperature of the heat sink 2404, an observation window 2408 used to observe the sample provided in the reaction chip container 2401, and a fluorescence detection module 2409 used for fluorescence observation, through the observation window 2408, of the state of the sample in the sample liquid.

For the DNA reaction chip container 2401, the structure of the reaction container or the optical fluorescence detection system used in the liquid reflux type or optical absorption heating type reaction control device described above is usable with no modification. Regarding the heat source, the heat sink 2404 controlled to have the DNA elongation reaction temperature (middle temperature) is used as a stable heat source. Heat is supplied from this heat sink to the DNA reaction chip via the Peltier element 2403 and the heat conductive thin plate 2402 (formed of a highly conductive metal material such as aluminum, duralumin, gold, silver, tungsten, Nepal brass, magnesium, molybdenum, beryllium, and an alloy containing such a metal element) that transfers heat uniformly across a plane. This can raise the temperature of the DNA reaction chip to the DNA denaturation temperature (high temperature) or can lower the temperature of the DNA reaction chip to the annealing temperature (low temperature), at high speed. The temperature sensor 2406 is located on a top surface of the heat conductive thin plate 2402, which is located immediately below the reaction chip, and can monitor the temperature of the reaction chip on the top surface of the heat conductive plate in real time. Thus, the temperature sensor 2406 can drive the Peltier element 2403 such that the measured temperature matches the temperature in a time-wise temperature change profile written in a program in advance. The heat sink is formed of a material such as a metal material or the like that has a sufficient heat capacity and a sufficient heat conductivity to be stable the middle temperature (e.g., aluminum, duralumin, gold, silver, tungsten, Nepal brass, magnesium, molybdenum, beryllium, or an alloy containing such a metal element). The temperature of the heat sink is monitored by a module that can supply heat stably through the heating wire 2405 or the like by using the temperature sensor 2407 supplied with the heat sink. Based on the information from the sensor 2407, the temperature control module (e.g., feedback control mechanism) (the temperature control module has, for an electric current-driven heat source such as a heating wire or the like, a function of controlling the quantity and the polarity of the electric current, and for a liquid reflux type temperature controller, a function of transmitting an ON/OFF control signal to a water temperature heating/cooling module) supplies necessary heat to the heat sink constantly, so that the heat sink is kept at the middle temperature. A significant feature of the present invention is that in the state where the heat sink is set to have the middle temperature (note: strictly, in order to set the temperature of the reaction vessel or the reaction container to the middle temperature, the temperature of the heat sink may be possibly shifted to the high temperature side or the low temperature side to some extent from the set temperature of the reaction vessel, in consideration of the loss during the heat conduction), when the Peltier element is turned off, the reaction control device can adjust the temperature of the DNA reaction chip to the intended DNA elongation reaction temperature, which is the middle temperature, as an equilibrium temperature. The reaction control device can perform such adjustment autonomically and at high speed with no overshoot. In general, the technique of maintaining the temperature at a certain level by continuously supplying heat by use of a feedback control element such as a Peltier element or the like has problems, for example, that overshoot is likely to be caused as a result of the feedback control, that the temperature is likely to fluctuate, that the power consumption is significantly increased, and the continuous operation of the Peltier element shortens the life thereof. By contrast, according to the present invention, the middle temperature is realized by turning off the Peltier element. The Peltier element is allowed to be turned off during the PCR elongation reaction, which is most time-consuming among various steps of PCR reaction. In addition, the elongation reaction temperature can be realized while the temperature approaches the equilibrium point, notably with no risk that the double strands of the DNA, which are bonded together by annealing performed at the low temperature, are separated from each other by the overshoot, which is caused by temperature rise. The DNA denaturation temperature on the high temperature side and the annealing temperature on the low temperature side do not need to be precisely controlled because the Peltier element is operated for a short time of about 1 second (denaturation reaction and annealing reaction) and slight overshoot is tolerated. In addition, the heat source in contact with the Peltier element, which supplies the heat to the DNA reaction chip and deprives the DNA reaction chip of the heat, is stable at the middle temperature. Therefore, the quantity of the electric current that is needed in order to raise the temperature of the DNA reaction chip to an intended level can be generally estimated. As described above regarding the liquid reflux type device, the observation window 2408 provided with a heat-generating transparent electrode formed of ITO or the like is located in the top surface of the DNA reaction container 2401, and the heat-generating transparent electrode supplies heat such that the temperature of the top surface of the chip is 75°C or higher. Owing to this, evaporation of the reagent in the chip from a bottom surface thereof and dew condensation on the top surface of the container can be prevented. Even when a trace amount of reagent reaction solution does not evaporate and the PCR reaction thereof can be detected by the fluorescence detection module 2409.

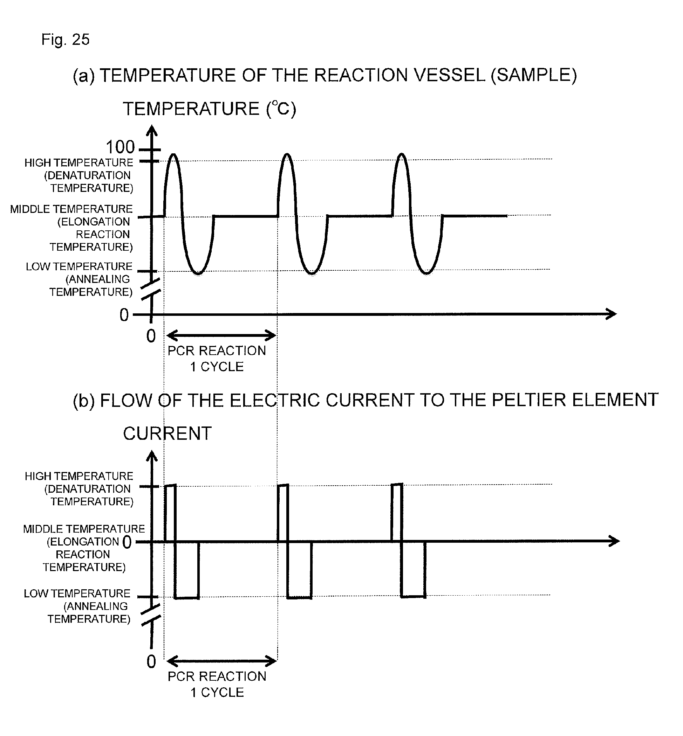

FIG. 25 schematically shows (a) the actual reaction temperature of the reagent in the DNA reaction chip and (b) an example of application of the electric current to the Peltier element. As can be seen from the graphs, the operation time of the Peltier element is limited to a short time that is required for transition to the high temperature or to the low temperature. After being lowered to the low temperature, the temperature is raised to the middle temperature by thermal equilibrium caused by heat conduction from the heat sink to the DNA reaction chip.

According to the present invention, the temperature can be controlled so as to be changed at high speed. Therefore, in the case where, for example, the denaturation temperature (high temperature) is set to 94°C, the elongation temperature (middle temperature) is set to 72°C and the annealing temperature (low temperature) is set to 55°C for a standard PCR, the time required for each of temperature changes from the middle temperature to the high temperature, from the high temperature to the middle temperature, from the middle temperature to the low temperature, and from the low temperature to the middle temperature can be a short time of, for example, 2 seconds, 1 second, 0.9 seconds, 0.8 seconds, 0.7 seconds, 0.6 seconds, 0.5 seconds, 0.4 seconds, 0.3 seconds or a shorter time.

In this example, a three-temperature PCR is described. A two-temperature PCR using only the high temperature and the middle temperature can be realized in a similar manner by merely turning on or off the Peltier element for the high temperature. The melting curve analysis and the RT-PCR process can be realized by slowly changing the temperature of the heat sink, or by performing feedback control on the heat supply from the Peltier element to the DNA reaction chip by use of the temperature sensor such that the temperature is slowly changed.

In this example, the heat sink and the heating wire are used as the heat source for the middle temperature. Alternatively, in a high-speed liquid reflux type reaction control device including one liquid reservoir set to have the middle temperature or in a high-speed liquid reflux type reaction control device usable for a two-temperature or three-temperature PCR as shown in FIG. 1, FIG. 13 or FIG. 21, one liquid reservoir set to have the middle temperature and the heat exchange chamber 3 may be used instead of the heat sink. In a liquid reflux type reaction control device that includes a plurality of reservoirs and thus is usable for a two-temperature or three-temperature PCR, a variety of temperatures can be advantageously used as the middle temperature.

### INDUSTRIAL APPLICABILITY

The present invention is useful as a reaction device for carrying out a reaction that requires strict control on the temperature of a sample. The present invention is also useful as a reaction device for carrying out a reaction that requires rapid change of the temperature of a sample.

In particular, the present invention is useful as a PCR device capable of carrying out a PCR at high speed, high precision and high amplification rate. A device of the present invention can be downsized, and is also useful as a portable PCR device.

### REFERENCE SIGNS LIST

- 1: Reaction vessel
- 2: Reaction vessel casing
- 3: Heat exchange chamber
- 4: Liquid reservoir tank
- 5: Heat source
- 6: Stirring mechanism
- 7: Pump
- 8: Switching valve
- 9: Bypass flow channel
- 90: Joint
- 10: Auxiliary temperature control mechanism
- 11: Inlet A
- 12: Inlet B
- 13: Outlet A
- 14: Outlet B
- 15: Coupling tube
- 16: Temperature sensor
- 17: Auxiliary liquid heat release mechanism
- 18: Direction of liquid flow
- 19: Peltier temperature control mechanism
- 20: Flow channel tube for liquid
- 2001: Air inlet tube
- 2002: Discharge tube for liquid in the heat exchange chamber
- 2003: Pressure leak valve
- 21, 22, 23, 24, 26, 231: Reaction vessel
- 25: Lyophilized reagent
- 27: Dispensing chip
- 28: Sample
- 29: Fiber ball
- 31: Reaction vessel
- 32: Reaction vessel casing
- 33: Reaction vessel socket
- 34: Thread
- 35: Seal
- 36: Tapered reaction vessel casing
- 37, 38: Heat exchange chamber
- 41: Inlet valve A
- 42: Outlet valve A
- 43: Inlet valve B
- 44: Outlet valve B
- 51: Glass-slide like reaction vessel casing
- 52, 58: Reaction vessel socket of the heat exchange chamber
- 53: Guide rail
- 54: Seal
- 55: Slide socket
- 56: Hinge
- 59: Reaction vessel
- 61: Inlet A
- 62: Outlet A
- 63: Inlet B
- 64: Outlet B
- 65: Piston
- 66: Reaction vessel
- 67: Heat exchange chamber
- 71: Piston
- 72: Piston rod
- 73: Piston
- 74: Magnet
- 75: Electromagnetic coil
- 76: Piston
- 81: Rotary valve
- 82: Rotation shaft
- 83: Heat exchange chamber
- 84: Reaction vessel
- 91: Inlet A
- 92: Outlet A
- 93: Inlet B
- 94: Outlet B
- 95: Membrane A
- 96: Membrane B
- 97: Reaction vessel
- 98: Heat exchange chamber
- 101: Rotary valve
- 102: Groove
- 103: Heat exchange chamber
- 104: Inlet A
- 105: Outlet A
- 106: Inlet B
- 107: Outlet B
- 108: Flow channel
- 109: Reaction vessel
- 110: Temperature
- 111: Elapsed time
- 201: Fluorescence detector
- 202: Control analyzer
- 203: Control signal
- 204: Optical window
- 1010: O-ring
- 1011: Guide rail
- 1012: Reverse transcription reaction temperature plate
- 1013: Glass plate
- 1014: Transparent electrode
- 1015: Reaction vessel
- 1016: Reverse transcription reaction vessel part
- 1101: Reaction vessel
- 1102: Reaction well
- 1201: Fluorescence detection probe
- 1202: Scanning direction for fluorescence detection probe
- 1203: Arrayed fluorescence detection probe
- 1301: Pillar
- 1302: Seal for preventing evaporation
- 1303: PCR solution
- 1304: Intercalator
- 1305: Fluorescence probe
- 1306: DNA probe
- 1401: Micro flow channel type reaction vessel
- 1402: Micro flow channel
- 1403: Flow channel-forming polymer
- 1404: Reaction vessel
- 1405: Sample injection opening
- 1406: Air reservoir for sample recovery
- 1407: Reaction liquid reservoir
- 1408: Sample discharge opening
- 1411: Syringe pump
- 1501: Illumination light source (halogen lamp, etc.)
- 1502: Condenser lens
- 1503: Automatic XY stage
- 1504: X-axis motor
- 1505: Y-axis motor
- 1506: Stage heater
- 1507: Reaction well plate
- 1508: Objective lens
- 1509: Infrared laser dichroic mirror
- 1510: Infrared laser
- 1511: Beam expander
- 1512: Laser shutter
- 1513: Motor (stepping motor, etc.)
- 1514: Shaft
- 1515: Gradation ND filter
- 1516: Fluorescence-exciting light dichroic mirror
- 1517: Fluorescence-exciting light source (mercury lamp, etc.)
- 1518: Fluorescence-exciting light source shutter
- 1519: Fluorescence-exciting light transmitter lens
- 1520: Camera dichroic mirror
- 1521: Imaging lens
- 1522: Image observation camera (cooled CCD camera, etc.)
- 2401: DNA reaction chip container
- 2402: Heat conductive thin plate
- 2403: Peltier element
- 2404: Heat sink
- 2405: Heating wire
- 2406, 2407: Temperature sensor
- 2408: Observation window
- 2409: Fluorescence detection module

## Claims

1. A device for gene analysis or gene amplification, the device comprising:
a reaction vessel including one or a plurality of wells for accommodating a sample liquid containing nucleic acid;
a heat sink or a heat exchange chamber;
a temperature controller, for the heat sink or the heat exchange chamber, that keeps a temperature of the heat sink or the heat exchange chamber at a first temperature;
a thermoelectric element that is located between the heat sink or the heat exchange chamber and the reaction vessel and acts as a medium for heat transfer between the heat sink or the heat exchange chamber and the reaction vessel; and
a thermoelectric element controller that changes a temperature of the reaction vessel between the first temperature and a second temperature by controlling an operation of the thermoelectric element;
wherein when the thermoelectric element is in an off state, the temperature of the reaction vessel is the first temperature.

2. The device according to claim 1, wherein:
the first temperature is a nucleic acid elongation reaction temperature; and
the second temperature is (i) a nucleic acid denaturation temperature or (ii) the nucleic acid denaturation temperature or a nucleic acid annealing temperature.

3. The device according to claim 1 or 2, wherein the temperature controller includes:
(i) a temperature sensor;
(ii) a heating wire for heating the heat sink, and
(iii) a feedback control mechanism that controls power supply to the heating wire based on temperature information on the heat sink from the temperature sensor.

4. The device according to claim 1 or 2, wherein the temperature controller is a liquid reflux type temperature controller that controls the temperature of the heat exchange chamber by refluxing a liquid of a predetermined temperature between the heat exchange chamber and a liquid reservoir that is in fluid connection with the heat exchange chamber by a tubular flow channel and holds the liquid of the predetermined temperature.

5. The device according to any one of claims 1 through 4, wherein the thermoelectric element controller includes:
a temperature sensor; and
a computer that controls an operation of the thermoelectric element based on temperature information on the reaction vessel from the temperature sensor.

6. The device according to any one of claims 1 through 5, wherein the thermoelectric clement is a Peltier element.

7. The device according to any one of claims 1 through 6, further comprising a heat conductive thin plate located between the thermoelectric element and the reaction vessel in order to raise a heat transfer efficiency between the thermoelectric element and the reaction vessel.

8. The device according to any one of claims 1 through 7, wherein a bottom surface and a wall surface of the reaction vessel each have a thickness of 1 micrometer to 100 micrometers and are formed of a metal material selected from the group consisting of aluminum, nickel, magnesium, titanium, platinum, gold, silver and copper, or silicon.

9. The device according to any one of claims 1 through 8, wherein an amount of the sample liquid is several tens of microliters per well.

10. The device according to any one of claims 1 through 9, further comprising a fluorescence detector that, in the case where the sample liquid contains a fluorescence dye, detects fluorescence emitted by the fluorescent dye in the one or the plurality of wells in association with a switch of the temperature of the reaction vessel, and measures a time-wise change in fluorescence intensity.

11. The device according to any one of claims 1 through 10, further comprising a reaction vessel casing that covers the reaction vessel in order to prevent evaporation of droplets of the sample liquid located in the one or the plurality of wells and includes a heat retention member for preventing dew condensation.

12. The device according to claim11, wherein the reaction vessel casing further includes an aperture or an optical window that facilitates measurement of an optical signal from the sample liquid in the reaction vessel casing; and the optical window is provided with an optically transparent heat generator.

13. The device according to any one of claims 1 through 9, wherein a pillar is located at a position where the sample liquid is located in each of the one or the plurality of wells in the reaction vessel; the one or the plurality of wells are each covered with a sealant for preventing evaporation of the sample liquid such that the sealant is supported by the pillar; and the pillar prevents the sample liquid under measurement from being attached to the sealant for preventing evaporation of the sample liquid.

14. A method for performing a PCR by use of the device according to any one of claims 1 through 13, the method comprising the steps of:
(a) locating a sample liquid containing nucleic acid in a well;
(b) switching a temperature of a reaction vessel from a nucleic acid elongation reaction temperature as a first temperature to (i) a nucleic acid denaturation temperature or (ii) the nucleic acid denaturation temperature or a nucleic acid annealing temperature as a second temperature;
(c) continuously switching the temperature of the reaction vessel from the second temperature to the first temperature; and
(d) repeating the step (b) and the step (c) a predetermined times at a predetermined time interval;
wherein the switch of the temperature of the reaction vessel from the second temperature to the first temperature is performed by turning off an operation of a thermoelectric element.

15. The method according to claim 14, wherein in the step (d), repeating the step (b)(ii) and the step (c) the predetermined times includes repeating, at a predetermined time interval, alternate performance of:
switching the temperature of the reaction vessel from the nucleic acid elongation reaction temperature as the first temperature to (b)(ii) the nucleic acid denaturation temperature as the second temperature and continuously switching the temperature of the reaction vessel from the nucleic acid denaturation temperature to the first temperature; and
switching the temperature of the reaction vessel from the nucleic acid elongation reaction temperature as the first temperature to (b)(ii) the nucleic acid annealing temperature as the second temperature and continuously switching the temperature of the reaction vessel from the nucleic acid annealing temperature to the first temperature.
